# EUROPEAN PATENT SPECIFICATION

(11) **EP 2 413 911 B1**
(45) Date of publication and mention of the grant of the patent: **19.02.2014**
(21) Application number: 10712359.8
(22) Date of filing: 31.03.2010
(51) Int. Cl.: A61K 9/16, C07D 215/18

(54) **PROGRESSIVE EMULSION CRYSTALLIZATION**
PROGRESSIVE EMULSIONSKRISTALLISATION
CRISTALLISATION PROGRESSIVE EN ÉMULSION

(30) Priority: 31.03.2009 SI 200900087
(43) Date of publication of application: 08.02.2012
(73) Proprietor: KRKA, D.D., Novo Mesto, 8501 Novo mesto (SI)
(72) Inventor: KLJAJIC, Alen, 3000 Celje (SI); ZUPEZ, Rok, 1000 Ljubljana (SI)
(74) Representative: Andrae Westendorp
(86) International application number: PCT/EP2010/002078
(87) International publication number: WO 2010/112222

(56) References cited:
- WO-A1-99/12623
- US-A- 5 872 259
- US-A1- 2004 197 275
- US-A1- 2007 213 365
- US-B1- 6 855 176

## Description

### FIELD OF THE INVENTION

The invention described herein is in the field of separation processes, more particularly, in the field of selective crystallization methods for purification of organic substances.

### BACKGROUND OF THE INVENTION

Solvent emulsion crystallization using two immiscible liquids and optionally surface active agents (surfactant)/co-surfactants forming emulsion (usually droplet type) is known in the art. In this type of crystallization systems, the mixture of chemical substances is dissolved mainly in one phase (dispersed phase), acting as a solvent phase and forming an emulsion with another immiscible liquid phase, anti-solvent phase. At higher temperatures, after the mixture of compounds has been dissolved, the emulsion is cooled to a targeted temperature where crystallization process takes place. After the crystallization is completed formed crystals are isolated from the emulsion (I. Holeci, Chem.Prumysl, 14, 145, 1964).
Instead of the solvent phase, the solute alone can present the immiscible phase in emulsion system (R. J. Davey, J. Garside, A. M. Hilton, D. McEwan, J. W. Morrison, Letters to Nature, 357, 1995).

Using emulsion media for crystallization (melt emulsions, solvent emulsions) as selective separation process is known in the art. The same emulsion can be used several times as recycled solvent/anti-solvent media. It means that after the crystallization out of emulsion is completed the emulsion can be heated again and additionally dissolve the mixture of chemical substance (with the desired compound) and perform the emulsion crystallization step again (US 6,855,176). Although, as the impurities concentrate in the emulsion the separation potential becomes lower in each additional use of the crystallization media. Furthermore, microemulsions have been used for isolating one or more enantiomer components from a mixture of enantiomers through co-crystallization (US 6,570,036; WO 97/32644).

Crystallization of substances using thermodynamically stable micro-emulsions as crystallization media (Gibbs free enthalpy of formation ΔG < 0) is known (WO 97/32644). The typical micro-emulsion region of a continuous isotropic liquid area can be presented in a ternary phase diagram and usually with fairly large amount of surfactants and a co-surfactant (P. Kumar, K.L. Mittal; Handbook of microemulsion science and technology).
These types of emulsion are not always suitable for industrial crystallization due to low capacity, low yields (separation of diastereisomers), long crystallization times and high surfactant content in the emulsion (US 6,855,176; WO 97/32644).

As known in the art it is possible to operate with thermodynamically stable (micro)emulsions, dissolving the aggregate mixture at higher temperatures, cooling the emulsion to lower temperature and gaining a high level of super saturation. At this lower crystallization temperature, seeding is used to promote crystallization (WO 99/12623). This type of crystallization is characterized in seeding the desired component and seed crystals of at least one other substance. As the authors of WO 99/12623 have pointed out; it needs to be managed that no solid particles are in the crystallization container because they might promote crystallization of the impure substance in combination with consecutive or simultaneous seeding. This is the critical point of the process and can have serious drawbacks for the large-scale industrial crystallization process.

There is a need to have a productive purification process, including preparation, separation and isolation of pure materials, appropriate for large-scale production of pure organic molecules, starting from crude materials. Especially, whenever the crude materials include related impurities which have similar structure and properties as the parent material and are hard to purify using classical purification techniques. The need is most important in the field of synthesis of fine chemicals, more precisely chemicals, which are intermediates in the production of active pharmaceutical ingredients and the pharmaceutical ingredients themselves. There thus exists a need in the art for efficient emulsion crystallization as purifying process using any kind of emulsions not limited to emulsion stability and having easy and simple operating process with fast crystallization times, large capacity of process, high yields, efficient separation and low amount of surfactant needed. Also, there is a need in the art to manipulate the filtration properties of crystallized products from emulsion.

We have developed the Progressive Emulsion Crystallization (PEC) to overcome these difficulties. Its characteristics are high yields and good separation of related impurities which is used for obtaining a highly pure product with good filtration properties, appropriate for use with all types of droplet emulsions.

### SUMMARY OF THE INVENTION

The present invention provides a benefit of progressive emulsion crystallization operating process in a batch, close to continuous or continuous operating way with the use of an appropriate homogenization system to keep suspension, and/or emulsion dispersed. Using the developed crystallization operating system enables the use of wide-range ratios of organic liquid/water/surfactant to control the process according to the properties of the impure substance and also to have a minimal amount of surfactant added and still have a good purification potential. This is needed especially when operating with active pharmaceutical ingredients and their intermediates.

The authors have developed an operating system, which enables efficient purification of chemical substances using droplet emulsions with unrestricted droplet size. The developed crystallization operating system in a reactor with appropriate homogenization enables the use of emulsions, characterized in Free Gibbs energy of droplet formation ΔG > 0, as shown in the examples.

We developed two basic principles for the application of PEC:
a.) The principle of purification of impurities, in particular related impurities from crude products of preferably organic molecules. The process is characterized in that the mixture of compounds is dissolved at high temperatures, the prepared emulsion is preferably cooled to an optimized temperature between the temperature at which the mixture of compounds is dissolved in the emulsion and the temperature at which the crystallization is completed, and the seeds of the desired component are added. The temperature of the crystallization medium is cooled to the target temperature, preferably gradually, in particular with an optimized cooling ramp. After the targeted temperature is achieved, preferably the suspo-emulsion is continued to be homogenized for some period of time. Finally, the product formed is isolated. This emulsion purification processes have high overall yields and purification potentials and are appropriate for large-scale, industrial applications.
b.) The principle of design of physical properties of materials: After the crystallization out of emulsion is completed, particles of the product with good filtration properties and appropriate for large-scale production are required. The authors of the present invention have found out that the use of the processes of the present invention produces purified materials with good filtration properties, which are needed for large-scale production. This is mainly because of larger particles formed due to an optimized control of emulsion crystallization, PEC.

Also, the authors of the present invention have surprisingly found out that agglomeration of particles takes place, if, preferably at the end of the crystallization process, an additional amount of the emulsion is added to the crystallization mixture, said crystallization mixture being for example a suspo-emulsion formed after seeding and/or during crystallization, instead of additional amounts of the continuous or droplet phase from which the compound crystallizes.

### BRIEF DESCRIPTION OF THE FIGURES

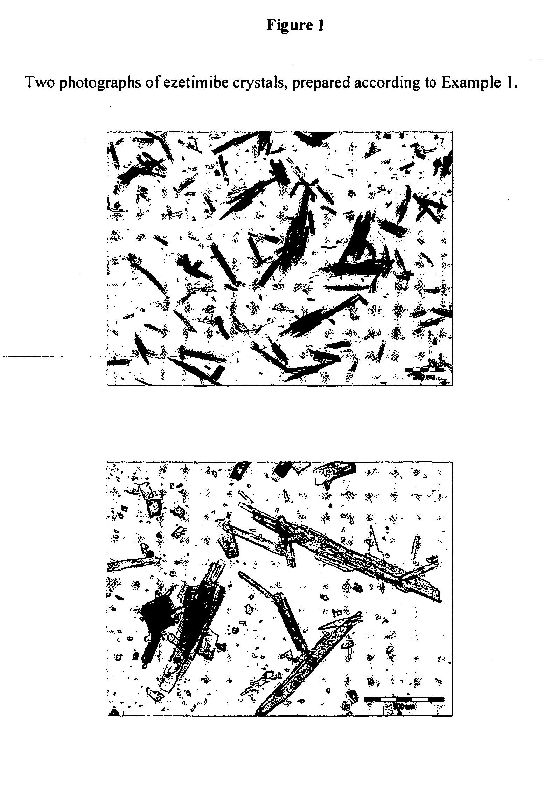

Fig. 1 shows two photographs of ezetimibe crystals, prepared according to Example 1.
Fig. 2 shows two photographs of fipronil crystals, prepared according to Example 2.
Fig. 3 shows two photographs of fipronil crystals, prepared according to Comparative Example 2.
Fig. 4 shows two photographs of montelukast acid crystals, prepared according to Example 3.
Fig. 5 shows two photographs of orlistat crystals, prepared according to Example 4.
Fig. 6 shows two photographs of orlistat crystals, prepared according to Comparative Example 4.
Fig. 7 shows two photographs of orlistat crystal agglomerates, prepared according to Example 4/a with addition of emulsion to the crystalllization system after the crystallization is performed.
Fig. 8 shows two photographs of (3R,5R)-tert-butyl 7-(2-(4-fluorophenyl)-5-isopropyl-3-phenyl-4-(phenylcarbamoyl)-1*H*-pyrrol-1-yl)-3,5-dihydroxyheptanoate crystals, prepared according to Example 5.
Fig. 9 shows two photographs of trityl candesartan cilexetil crystals, prepared according to Example 7.
Fig. 10 shows two photographs of trityl candesartan cilexetil crystals, prepared according to Comparative Example 7.
Fig. 11 shows two photographs of orlistat crystals, prepared according to Example 10.
Fig. 12 shows two photographs of orlistat crystals, prepared according to Comparative Example 10.
Fig. 13 shows two photographs of montelukast acid crystals, prepared according to Example 11.
Fig. 14 shows two photographs of montelukast acid crystals, prepared according to Comparative Example 11.

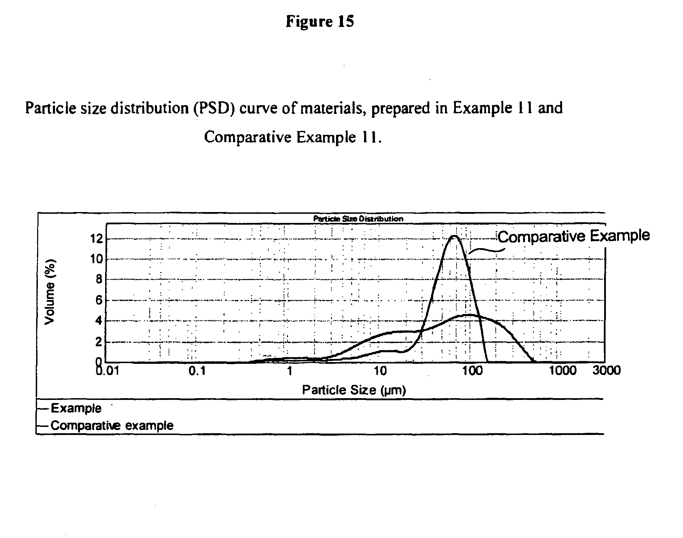

Fig.15 shows the particle size distribution (PSD) curve of materials, prepared in Example 11 and Comparative Example 11.

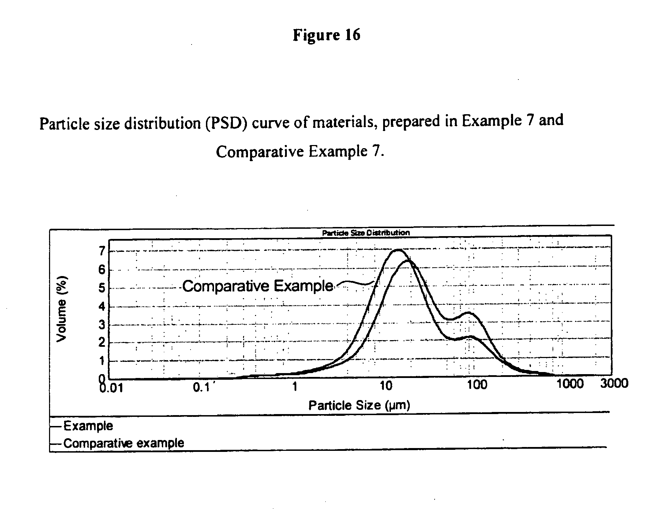

Fig. 16 shows the particle size distribution (PSD) curve of materials, prepared in Example 7 and Comparative Example 7.

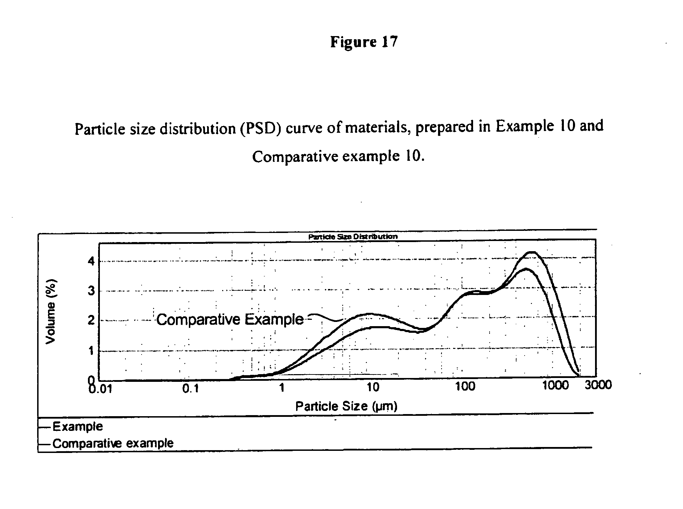

Fig. 17 shows the particle size distribution (PSD) curve of materials, prepared in Example 10 and Comparative example 10.

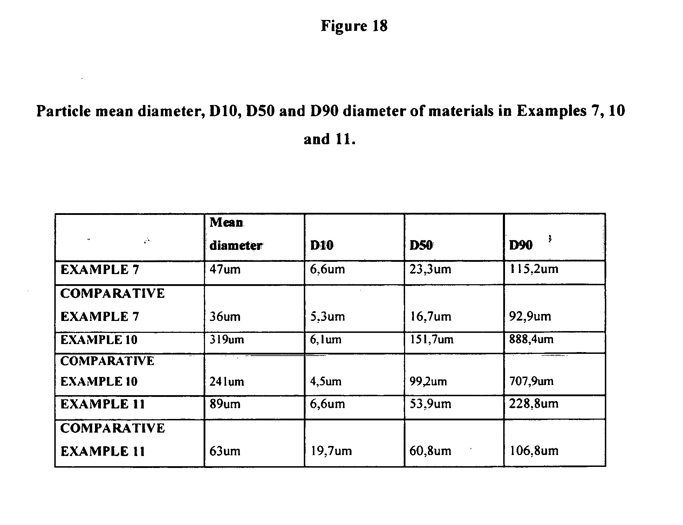

Fig. 18 shows the particle mean diameter, D10, D50 and D90 diameter of materials in Examples 7, 10 and 11.

### DETAILED DESCRIPTION OF THE INVENTION

According to a first aspect, the present invention provides a crystallization process for the preparation of chemical compounds, in particular pure chemical compounds, in which an emulsion of droplets in a continuous phase is formed having Gibbs free enthalpy of formation ΔG>0, to effect crystallization and purification of the crude substance in the continuous or droplet phase, in which process seeding with crystals of the pure substance is carried out at a higher temperature than the temperature at which the crystallization is completed and in which process the emulsion comprises a surface active agent.

The term "crystallization process for the preparation of chemical compounds, in particular pure chemical compounds" may encompass in particular processes wherein the substance obtained after having carried out the crystallization process comprises an amount (percentage of weight) of the respective chemical compound being the target component, which amount (percentage of weight) is higher than the amount (percentage of weight) of the chemical compound being the target component present in the crude substance before carrying out the crystallization process.
The term "seeding with crystals of the pure substance" may encompass in particular a seeding with crystals comprising an amount (percentage of weight) of the respective chemical compound being the target component, which amount (percentage of weight) is higher than the amount (percentage of weight) of the respective chemical compound being the target component present in the crude substance. For example, the crystals used for seeding can have a purity of above 99 % (w/w), in particular of above 99.5 % (w/w), further in particular of at least 99.8 % (w/w), especially of 100 % (w/w).

The term "the temperature at which the crystallization is completed" may encompass for example the temperature at which no more or essentially no more crystals precipitate, or e.g. the temperature, at which the crystal precipitation is stopped or interrupted, in particular before starting the isolation of the crystals resulting from the crystallization process of the present invention. Preferably the crystal precipitation is stopped or interrupted when no more or essentially no more crystals precipitate.

The term "surface active agent" is known to a person skilled in the art and may encompass for example agents lowering the surface tension of a liquid and in particular may encompass agents lowering the surface tension of one or more of the components of the emulsion used during the crystallization process of the present invention.

Formation of emulsions is well-known in the art and is by definition "droplets" dispersed in a "continuous phase". Optionally, the emulsions contain additives such as surface active agents lowering the interfacial tension by adsorption of surfactants and stabilizing the emulsion droplets to a certain degree (Eli Ruckenstein, J. C. Chu; Stability of Microemulsions, J Chem Soc Faraday Trans II 1975;71:1690-1707). The droplet size is not limited and usually in the range between 0.2-100 µm.

Solvents which may be used in combination with water (in the droplets or in the continuous phase) are non-polar, lipophilic solvents as n-, i- or branched having formula CₙH₂ₙ₊₂ (alkanes), CₙH₂ₙ (alkenes), CₙH₂ₙ₋₂ (alkynes), aromatics, halogenated hydrocarbons, ethers, aldehydes, natural or mineral oils, ketones, esters, amides, etc. An appropriate solvent can be any solvent which is at least partially immiscible with water and consequently capable of forming an emulsion with water. One, two or more solvents can be used with water simultaneously.

Water insoluble solvents are all solvents which are not miscible with water or have very low solubility in water, and thus can form an emulsion with water. The dielectric constant of the solvent used as the organic phase is below 15 and is not limited for co-solvents, if the latter are used together with a first solvent.

One of the phases is thus comprised by water, to which other, water soluble polar solvents may be admixed, and the second phase is comprised by one or more unpolar solvents, which are not soluble in water or its mixtures with water soluble polar solvents. Usually the droplet is formed by the organic lipophilic solvent and the continuous phase comprises water.

All emulsions used are characterized by showing free Gibbs energy of droplet formation ΔG > 0, which means that if this emulsions are left to stand without homogenization, the liquid phases are separated after a certain period of time. Emulsions showing free Gibbs energy of droplet formation lower than 0, on the contrary, remain unchanged even if not constantly homogenized once they are formed.

The target component may theoretically crystallize or precipitate either from the droplet or the continuous phase but in most cases the first case occurs.

Usually the droplet phase of the emulsion will contain the aggregate mixture to be purified and may optionally contain one, two or more solvents. Surprisingly, it was found that the desired substance may also be practically insoluble in the dispersed "droplet" phase when compared to the continuous phase, leading to the precipitation or crystallization of the target component from the continuous phase.

Furthermore, the emulsion can contain a buffering agent and/or solubility adjusting agents, as known in the art.

The emulsion according to the present invention contains one or more surface active agents and/or dispersants which assist in forming an emulsion. The surface active agents can be non-ionic, cationic or anionic, as known in the art. For example, the surfactant can be or can comprise a di(C₆-C₁₂ alkyl) sodium sulfosuccinate said C₆-C₁₂ alkyl being a straight chain or being a branched C₆-C₁₂ alkyl, such as dioctyl sodium sulfosuccinate, an ethoxylated polyarylphenol phosphate based surfactant, a poly(ethylene glycol)-*block-*poly(propylene glycol)-*block*-poly(ethylene glycol) tri-block co-polymer or mixtures thereof.

The purification process of the present invention is appropriate for crude/aggregate substances which contain at least 30% by weight of the target component, preferably more than 50% by weight of the target component, more preferably more than 70 % by weight of the target component.

The "target component" is a chemical compound of interest as such, in particular any organic molecule, preferably a or the pure pharmaceutically active compound or its intermediate, is meant.

The term "aggregate mixture" or "crude substance" is used when unsatisfactorily pure target components are meant, which contain an elevated level of impurities, either synthetic, biosynthetic or degradation impurities by their origin. The term "pure substance" means a substance having less impurities than a crude substance, i.e. having a higher degree of purity than the crude substance.

"Targeted crystallization reactor temperature" is the lowest end temperature during the crystallization process. This is usually the temperature of suspo-emulsion immediately before the isolation process is performed.

In the examples the Reactor Temperature (RT) versus time during the cooling process is presented as a linear function, but is not limited thereto. It is used as an average cooling rate for describing the approximate time needed for cooling the mixture in the reactor to the desired temperature. The real function of the reactor temperature versus time of crystallization process can be represented by any other continuous function.

The "temperature of seeding" is the temperature, at which the seeds of pure chemical substance are added.

This method of crystallization may be used for purifying any type of organic molecules, preferably prazoles (e.g. lansoprazole, pantaprazole, rabeprazole, omeprazole, esomeprazole), statins (e.g. rosuvastatin, fluvastatin, simvastatin, lovastatin, atorvastatin, and their intermediates, preferably the intermediates of atorvastatin, most preferably (3 R,5 R)-tert-butyl 7-(2-(4-fluorophenyl)-5-isopropyl-3-phenyl-4-(phenylcarbamoyl)-1*H-*pyrrol-1-yl)-3,5-dihydroxyheptanoate, ezetimibe, solifenacin or its pharmaceutically acceptable salts, orlistat or its intermediate lipstatin, fipronil, montelukast or its pharmaceutically acceptable salts, preferably alkaline or piperazine salts, most preferably montelukast sodium, montelukast trans-2,5-dimethylpiperazine and montelukast 2-methylpiperazine, sartans (such as valsartan, candesartan cilexetyl, olmesartan, telmisartan, irbesartan, eprosartan, preferably candesartan cilexetyl and trityl candesartan cilexetyl) and quetiapine. More preferably, the crystallization of the present invention is used for the purification of intermediates or crude drug products: ezetimibe, orlistat, fipronil, montelukast, atorvastatin, candesartan and pantoprazole.

The process can preferably be used for the purification of crude candesartan cilexetyl and its intermediates, most preferably trityl candesartan cilexetyl with a resulting purity more than 95 % in a single crystallization cycle, as well as for the purification of orlistat and montelukast acid with resulting purity more then 97% in a single crystallization cycle. Montelukast acid purified by the process of the present invention can e.g. be prepared by first forming a montelukast salt, preferably a montelukast piperazine salt and further converting it to montelukast free acid. Alternatively, montelukast salt, preferably montelukast piperazine salt, most preferably montelukast trans-2,5-dimethylpiperazine salt or montelukast 2-metylpiperazine salt, may also directly be purified by the process of the present invention. Montelukast free acid can eventually be converted to montelukast sodium salt, preferably with a purity of above 99.5 %.

Most preferably, the crystallization in the case of ezetimibe and its derivates is performed with phase inversion, characterized in that purified ezetimibe is crystallized from the continuous and not from the droplet phase.

The emulsions comprising two immiscible liquids, surfactants, and optionally other additives yield in one single crystallization cycle at least 50%, preferably 70%, and more preferably more than 85% of the purified target component. The emulsion is heated and the mixture of substances (or crude substance or aggregate mixture) is dissolved, the emulsion is cooled to the desired temperature (which is higher than the crystallization temperature) where the seeding is performed (Seeding Reactor Temperature), to induce the crystallization. The crystallization takes place within the time period of further lowering the temperature to the targeted crystallization temperature. In the present invention best results have been observed when:
- the final targeted crystallization reactor temperature at which the crystallization is completed, is 2 - 50°C below the seeding temperature, and
- the temperature of seeding was between 0 - 65 °C lower than the temperature at which all agglomerate mixture is dissolved. Preferably, the temperature of seeding was between 0.5 - 65 °C lower, in particular 2 - 65 °C lower than the temperature at which all agglomerate mixture is dissolved.

For efficient control of selectivity of emulsion crystallization processes characterized in having Gibbs free enthalpy of formation ΔG>0, preferably an optimized cooling ramp of crystallization media is used. Preferably the dynamics of cooling is less than 1.5 K/min, preferably less than 1 K/min, more preferably less than 0.5 K/min. The obtained product is preferably isolated and washed. If needed, the process is repeated. Instead of seeding any other method for forced nucleation can be used, as known in the art (ultrasound etc.).

The seed crystals applied in the process of the present invention may be prepared according to any process described in the prior art or known to the person skilled in the art. For ezetimibe seed crystals of ezetimibe may e.g. be prepared as described in WO 2008/089984; for fipronil, seed crystals may e.g. be prepared as described in WO 2007/069254 and WO 2008/055881; for montelukast acid, the seed crystals of montelukast acid used may be prepared as disclosed in US 2005/0187243; and e.g. for orlistat, seed crystals may be prepared as described in WO 2005/026140. For any compound to be purified by the present invention, any seed crystals of any polymorphic form can be used, preferably provided the seed crystals of the compound have a purity above 99.0 %, preferably above 99.5%. For pantoprazole the seed crystals can e.g. be prepared as disclosed in WO 2009/027533 and for (3R,5R)-tert-butyl 7-(2-(4-fluorophenyl)-5-isopropyl-3-phenyl-4-(phenylcarbamoyl)-1*H*-pyrrol-1-yl)-3,5-dihydroxyheptanoate as e.g. described in WO 2005/097742.

In a preferred embodiment of the invention, an emulsion with Free Gibbs energy of droplet formation ΔG>0 is formed, the aggregate mixture to be separated/ purified is dissolved at an elevated temperature, then the emulsion is cooled to the seeding reactor temperature, after which the temperature is still lowered to the target crystallization reactor temperature at which the crystallization is completed.

Using PEC results in high capacity of the crystallization process, high separation potential for related impurities, high yields of crystallization cycle, low amounts of surfactant needed and also produces large particles with improved filtration properties, which is needed especially in the "scale-up" development of an industrial process. There is a need in art for crystallization process characterized in effective and low-cost separation appropriate for large-scale production of pure organic substances.

Isolation of crystals from the emulsion can be carried out by any conventional means, such as filtration or centrifuge.
In particular the present invention provides the following items:
1.) A crystallization process for the preparation of chemical compounds, in particular of pure chemical compounds in which an emulsion of droplets in a continuous phase is formed having Gibbs free enthalpy of formation ΔG>0, to effect crystallization and purification of the crude substance in the continuous or droplet phase, characterized in that seeding with crystals of the pure substance is carried out at a higher temperature than the temperature at which the crystallization is completed and in that the emulsion comprises a surface active agent.
2.) A process according to item 1, characterized in that the purity of the crude substance is less than 90 %, preferably less than 80 %, most preferably less than 60% (w/w).
3.) A process according to any of items I or 2, where the final targeted temperature, at which the crystallization is completed, is 2-50 °C below the temperature of seeding.
4.) A process according to any of items 1 to 3, where the temperature of seeding is 0-65 °C lower then the temperature, at which all of aggregate mixture is dissolved.
5.) A process according to any of items 1 to 4, where the average cooling rate for cooling the crystallization mixture from temperature, at which the seeding is done, to the targeted temperature is below I K/min, preferably below 0.5 K/min.
6.) A process according to any of items I to 5, where emulsion comprises an C₁-C₅ acetate ester as an organic phase.
7.) A process according to any of items 1 to 6 characterized in that agglomeration of formed particles is induced by addition of the emulsion to the existing suspo-emulsion.
8.) The use of the process according to any of items 1 to 7 for the purification of a group of compounds, comprising statins like rosuvastatin, fluvastatin, simvastatin, lovastatin, atorvastatin, and their intermediates, like (3R,SR)-tert-butyl 7-(2-(4-fluorophenyl)-5-isopropyl-3-phenyl-4-(phenylcarbamoyl)-1*H*-pyrrol-1-yl)-3,5-dihydroxyheptanoate; sartans like valsartan, losartan, candesartan cilexetyl, olmesartan, telmisartan and irbesartan or their intermediates like trityl candesartan cilexetyl; prazoles like lansoprazole, pantoprazole, rabeprazole, omeprazole, or esomeprazole, ezetimibe, fipronil, montelukast acid or montelukast salts, like montelukast 2-methylpiperazine salt or 2-methylpiperazine salt; orlistat or its intermediate lipstatin, solifenacin base or solifenacin salts, and quetiapine; preferably orlistat, trityl candesartan cilexetyl and montelukast acid.
9.) The use of the process according to any of items I to 8 for the purification of crude candesartan cilexetyl, trityl candesartan cilexetyl or any intermediate of candesartan with a purity more than 95 % in a single crystallization step and a resulting purity more than 99 % after two crystallization steps.
10.) A process according to item 9 where purity of the starting material is below 90%, preferably below 50 %.
11.) A process according to any of items 1 to 8 for the purification of orlistat or its intermediate lipstatin with a resulting purity more then 97% in a single crystallization step and minimum resulting purity more than 98.5% after two consecutive crystallizations.
12.) A process according to item 11 where the purity of the starting material is below 95%, preferably below 87%.
13.) A process according to any of items I to 8 for the purification of montelukast acid with a resulting purity more than 97% in a single crystallization step and a resulting purity more then 99.0 % after three consecutive crystallizations.
14.) A process according to item 13 where the purity of the starting material is below 95%, preferably below 90%.
15.) A process according to any of items 13 and 14, characterized in that montelukast acid to be purified by the process is prepared by first forming a montelukast piperazine salt, preferably montelukast trans-2,5-dimethylpiperazine salt or montelukast 2-methylpiperazine salt and then converting it to montelukast acid.

The term "C₁-C₅ acetate ester" encompasses for example C₁-C₅ alkyl acetate esters, in particular methyl acetate ester, ethyl acetate ester, and straight chain or branched C₃-C₅ alkyl acetate esters, and for example C₂-C₅ alkenyl acetate esters, e.g. straight chain C₂-C₅ alkenyl acetate esters and branched C₃-C₅ alkenyl acetate esters.

The present invention is illustrated by the following Examples without being limited thereto. With the examples we demonstrate the advantage of PEC with the concentrations of selected impurities, process yields and particle size distributions. Nevertheless, the invention is not limited to the selected impurities but is appropriate for the purification of any molecules and any related impurity.

All emulsions used are characterized by showing free Gibbs energy of droplet formation ΔG > 0, which means that if this emulsions are left to stand without homogenization, the liquid phases are separated after a certain period of time. Emulsions showing free Gibbs energy of droplet formation lower than 0, on the contrary, remain unchanged even if not constantly homogenized.

In all examples mechanical stirring for appropriate homogenization of the (suspo-) emulsion was used during the described crystallization process in the reactor.

In all examples the mass of the starting material and the mass of the obtained purified product refers to the product, dried under vacuum and nitrogen flow and having loss on drying < I w/w %. If necessary, the drying time is prolonged until material having loss on drying < I w/w % is obtained.

In all examples the solvents used have a purity of more than 98.0 w/w %.

In all examples the given temperatures refer to the average temperature of the mixture in the reactor (RT) during the described step of the process.

The surface-active agents used in the examples are:
i) Aerosol® OT-B (Cytec Industries Inc.), which consists of 85 w/w % dioctyl sodium sulfosuccinate and 15 w/w % sodium benzoate,
ii) Pluronic® F127 (Sigma Aldrich Co.), which is a poly(ethylene glycol)-*block-*poly(propylene glycol)-*block*-poly(ethylene glycol) tri-block co-polymer, and
iii) Soprophor® FL (Rhodia), which is an ethoxylated polyarylphenol phosphate based surfactant.

The purity or impurity content % is expressed as chromatographic purity (in area %). The assay is expressed as weight % and is determined by the use of the external standard method.

The analytical methods for the chromatographic purity of target molecules were the following:

### a.) FIPRONIL, HPLC method

| | |
|---|---|
| **Column:** | X Bridge C-8 75 x 4.6mm, 2.5µm, |
| **Eluent A:** | 0.01M (NH₄)₂HPO₄ pH=6.0 |
| **Eluent B:** | acetonitrile |
| **Eluent C:** | methanol |

**Gradient:**

| Time (min) | Eluent A (%) | Eluent B (%) | Eluent C (%) |
|---|---|---|---|
| 0 | 60 | 20 | 20 |
| 25 | 10 | 45 | 45 |
| 30 | 10 | 45 | 45 |
| 32 | 60 | 20 | 20 |

| | |
|---|---|
| **Post time:** | 3 min |
| **Flow-rate:** | 1.0 mL/min |
| **Detection:** | UV, wavelength 280 nm |
| **Injection volume:** | 20 µl |
| **Column temperature:** | 25°C |
| **Diluent:** | acetonitrile : methanol : water (40 : 30 : 30, V:V:V) |

### b.) MONTELUKAST, HPLC method

| | |
|---|---|
| **Column:** | Eclipse Plus C 18, 50 × 4.6 mm, 1.8 µm particles, |
| **Eluent A:** | 0.005 M sodium dihydrogen phosphate : acetonitrile : triethylamine = 65 : 35 : 0.2, pH=6.5 |
| **Eluent B:** | acetonitrile : water= 90 : 10 |

### Gradient:

| Time (min) | % A | % B |
|---|---|---|
| 0 | 85 | 15 |
| 3 | 85 | 15 |
| 10 | 60 | 40 |
| 30 | 0 | 100 |
| 36 | 0 | 100 |
| 40 | 85 | 15 |

| | |
|---|---|
| **Post time:** | 3 min |
| **Flow-rate:** | 1.0 mL/min |
| **Detection:** | UV, 225 nm |
| **Injection volume:** | 5 µl |
| **Column temperature:** | 25°C |
| **Diluent:** | methanol |

### c.) EZETIMIB, HPLC method for stereoisomeric purity

| | |
|---|---|
| **Column:** | Chiralpak AD-H, 250 x 4.6 mm, 5 µm |
| **Mobile phase:** | solvent A hexane |
| | solvent B: ethanol : methanol in the ratio 75:25 |
| **Isocratic elution:** | solvent A : solvent B in the ratio 90 : 10 |
| **Column temperature:** | 25°C |
| **Flow rate:** | 1.5 ml/min |
| **Detection:** | UV, 230 nm |
| **Injection:** | 10 µl |
| **Diluent:** | ethanol acidified with acetic acid (0.2 ml of acetic acid into 0.5 l of ethanol, mix well) |

### d.) TRITYL CANDESARTAN CILEXETYL, HPLC method

| | |
|---|---|
| **Column:** | Zorbax Eclipse Plus C18, 50 × 4.6 mm, 1.8 µm particles, |
| **Eluent A:** | 0.01M NaH₂PO₄, pH 2.5 |
| **Eluent B:** | acetonitrile |

### Gradient:

| Time (min) | % A | % B |
|---|---|---|
| 0 | 55 | 45 |
| 13 | 5 | 95 |
| 18 | 5 | 95 |
| 19 | 55 | 45 |

| | |
|---|---|
| Post time: | 3 min |
| Flow-rate: | 1.0 mL/min |
| Detection: | UV, wavelength 225 nm |
| Injection volume: | 5 µl |
| Column temperature: | 30°C |
| Diluent: | acetonitrile : water (80 : 20, V:V) |

### e.) PANTOPRAZOLE, HPLC method

| | |
|---|---|
| **Column:** | Gemini C 18 150 x 4.6 mm i.d., 3 µm particles |
| **Column temperature:** | 35°C |

### Mobile phase: gradient elution

A: 0.0 1M phosphate buffer solution pH 6.5
B: mixture of solvents (acetonitrile : methanol = 30 : 70) (V:V)

| **Time (min)** | **%A** | **%B** |
|---|---|---|
| 0 | 67 | 33 |
| 3 | 65 | 35 |
| 30 | 45 | 55 |
| 38 | 10 | 90 |
| 40 | 10 | 90 |
| 42 | 67 | 33 |

| | |
|---|---|
| **Post time:** | 2 minutes |
| **Flow:** | about 1.0 mL/min |
| **Detection:** | UV, wavelength 280 nm |
| **Injection:** | 20 µl |
| **Temperature of samples:** | 8°C |
| **Diluent:** | water : acetonitrile: TEA = 60 : 40 : 1 (V/V/V), adjust the pH of the solvent to 10.0 with H₃PO₄. |

### f.) ORLISTAT, HPLC method

| | |
|---|---|
| **Column:** | Inertsil ODS3, 150 × 4.6 mm, 3 µm particles, |
| **Eluent A:** | 0,1% phosphoric acid |
| **Eluent B:** | acetonitrile |

### Gradient:

| time (min) | % A | % B |
|---|---|---|
| 0 | 75 | 25 |
| 4.5 | 40 | 60 |
| 5 | 40 | 60 |
| 20 | 25 | 75 |
| 48 | 9 | 91 |
| 50 | 1 | 99 |
| 75 | 1 | 99 |
| 76 | 75 | 25 |

| | |
|---|---|
| **Post time:** | 4 min |
| **Flow-rate:** | 1.2 mL/min |
| **Detection:** | UV, 200 nm |
| **Injection volume:** | 20 µl |
| **Column temperature:** | 45°C |
| **Diluent:** | acetonitrile |

### g.) (3R,5R)-tert-butyl 7-(2-(4-fluorophenyl)-5-isopropyl-3-phenyl-4-(phenylcarbamoyl)-1H-pyrrol-1-yl)-3,5-dihydroxyheptanoate, HPLC method

| | |
|---|---|
| **Column:** | Luna PFP 100A, 3 µm, 150 x 4.6 mm |
| **Mobile phase:** | solvent A 0.01M ammonium acetate pH 3.8 |
| | solvent B: acetonitrile : tetrahydrofurane in the ratio 95:5 |

| time (min) | % A | % B |
|---|---|---|
| 0 | 65 | 35 |
| 30 | 32 | 68 |
| 42 | 31 | 69 |
| 45 | 20 | 80 |
| 47 | 65 | 35 |

| | |
|---|---|
| ***Post run:*** | 3 min |
| ***Column temperature:*** | 20°C |
| ***Flow rate:*** | 1.0 mL/min |
| ***Detection:*** | UV, 248 nm |
| ***Injection:*** | 10 µl |
| ***Diluent:*** | mixture of acetonitrile and water (70:30) |

The term "particle size distribution" means a particle size frequency distribution that shows the percentage of particles (normalized in means of mass, volume or count) found in each size range and usually displayed in a histogram form.

The term "mean diameter", describes the arithmetic average volume particle diameter of the distribution.

The term "d90 value" means that at least 90% of the particles have a volume diameter less than the specified value. The same approach goes for the d50 and d10. The term "d50 value" means that at least 50% of the particles have a volume diameter less than the specified value. The term "d10 value" means that at least 10% of the particles have a volume diameter less than the specified value.

In the case of montelukast, the particle size was determined by laser light scattering on Malvern-Mastersizer Apparatus MS 2000 equipped with Hydro 2000S dispersion cell unit using vegetable oil as the dilution medium (RI=1,469). Particle absorption index was set to 1.

In the case of trityl candesartan cilexetyl, the particle size was determined by laser light scattering on Malvern-Mastersizer Apparatus MS 2000 equipped with Hydro 2000S dispersion cell unit using Isopar L as the dilution medium (R₁=1,432). Particle absorption index was set to 1.

In the case of orlistat, the particle size was determined by laser light scattering on Malvern-Mastersizer Apparatus MS 2000 equipped with Hydro G dispersion cell unit using deionised water as the dilution medium (RI=1,330). Particle absorption index was set to 1.

The invention is further illustrated by the following examples which demonstrate the used of PEC for the purification of certain organic compounds without being limited thereto. Parts or percentages are based on weight, unless specified otherwise.

### EXAMPLES

### EXAMPLE 1

### Purification of ezetimibe by PEC

0.5 g of crude ezetimibe (enantiomeric purity 93.2 %) with 6.8 % of the impurity 1, (3R,4S)-1-(4-fluorophenyl)-3-((R)-3-(4-fluorophenyl)-3-hydroxypropyl)-4-(4-hydroxyphenyl)azetidin-2-one, was added to an emulsion consisting of 5 mL of chlorobenzene, 0.25 mL of water, 0.4 mL of methanol and 0.015 g of Aerosol® OT-B (85 w/w % dioctyl sodium sulfosuccinate, 15 w/w % sodium benzoate). The suspo-emulsion was heated from room temperature to 95 °C with average heating ramp 1 K/min and maintained for all solid to dissolve. The mixture was maintained at this temperature for 10 minutes and cooled to 50 °C with average cooling ramp 1 K/min. At this point seeds of pure ezetimibe were added (10 mg) and the emulsion was slowly cooled to 35 °C with average cooling ramp 0.05 K/min and maintained at this temperature for additional 5 hours to allow ezetimibe to fully crystallize. The formed product was isolated by filtration and washed with 0.5 mL of water. 0.36 g of ezetimibe with enantiomeric purity 98.3 %, with 1.4 % of the impurity 1, was obtained.

**Table 1: HPLC analysis of crystallized material.**

| | ***Impurity 1 content (%)*** | ***Purity of crystallized material (%)*** | ***Microscopic pictures of formed particles*** |
|---|---|---|---|
| EXAMPLE 3 | 1.4 | 98.0 | Figure 1 |

### EXAMPLE 2

### Purification of fipronil by PEC

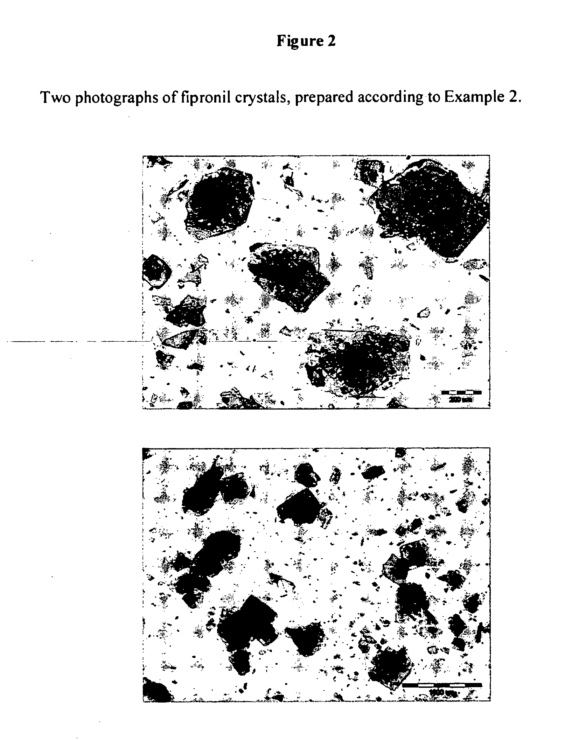

21g of crude fipronil (purity 94.2 %) with 4.4 % of the impurity 2, 5-amino-1-(2,6-dichloro-4-(trifluoromethyl)phenyl)-4-(trifluoromethylsulfonyl)-1*H*-pyrazole-3-carbonitrile, and 1.1 % of the impurity 3, 5-amino-1-(2,6-dichloro-4-(trifluoromethyl)phenyl)-4-(trifluoromethylthio)-1*H*-pyrazole-3-carbonitrile, was completely dissolved in an emulsion consisting of 9 mL of isopropanol, 25 mL of ethyl acetate, 30 mL of water and 2 g of Aerosol® OT-B (85 w/w % dioctyl sodium sulfosuccinate, 15 w/w % sodium benzoate). The suspo-emulsion was heated from room temperature to reflux with average heating ramp 1 K/min and maintained at this temperature for 30 minutes for all solid to dissolve. Furthermore, the emulsion was cooled to 40 °C and the seeds of pure fipronil were added (200 mg) at this temperature. Then, the emulsion was cooled to 0 °C with average cooling ramp 0.20 K/min and maintained for additional 2 hours to allow fipronil to fully crystallize. The formed product was isolated by filtration and washed with 15 mL of water.
14.6 g of product with 2.5 % of the impurity 2 and 0.50 % of the impurity 3, with 96.9 % fipronil purity, was obtained. The microscope pictures of formed product are shown in Figure 2.

### COMPARATIVE EXAMPLE 2

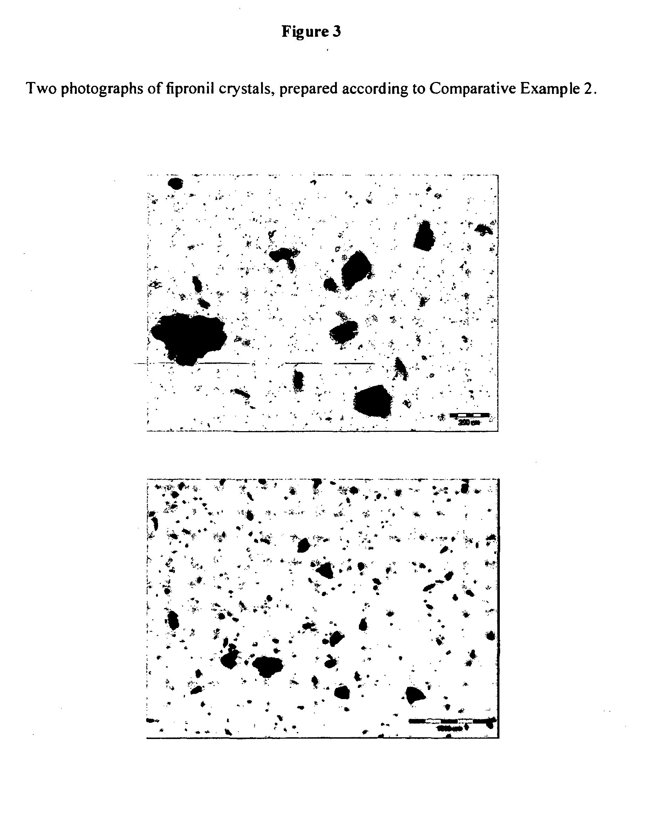

21g of crude fipronil (purity 94.2 %) with 4.4 % of the impurity 2, 5-amino-1-(2,6-dichloro-4-(trifluoromethyl)phenyl)-4-(trifluoromethylsulfonyl)-1*H*-pyrazole-3-carbonitrile, and 1.1 % of the impurity 3, 5-amino-1-(2,6-dichloro-4-(trifluoromethyl)phenyl)-4-(trifluoromethylthio)-1*H*-pyrazole-3-carbonitrile, was completely dissolved in an emulsion consisting of 9 mL of isopropanol, 25 mL of ethyl acetate, 30 mL of water and 2 g of Aerosol® OT-B (85 w/w % dioctyl sodium sulfosuccinate, 15 w/w % sodium benzoate). The suspo-emulsion was heated from room temperature to reflux with average heating ramp 1 K/min and maintained at this temperature for 30 minutes for all solid to dissolve. Furthermore, the emulsion was cooled to 0 °C and the seeds of pure fipronil were added (200 mg) at this temperature. The emulsion was maintained for additional 5 hours to allow fipronil to fully crystallize. The formed product was isolated by filtration and washed with 15 mL of water. The microscope pictures of formed product are presented in Figure 3.
14.3 g of 95,4 % fipronil product with 3.9 % of the impurity 2 and 0,6 % of the impurity 3 was obtained.

**Table 2: HPLC analysis of crystallized materials in Example 2 and Comparative Example 2.**

| | ***Impurity 2 content (%)*** | ***Impurity 3 content (%)*** | ***Purity of crystallized material (%)*** | ***Microscopic pictures of formed particles*** |
|---|---|---|---|---|
| EXAMPLE 2 | 2.5 | 0.5 | 96.9 | Figure 2 |
| COMPARATIVE EXAMPLE 2 | 3.9 | 0.6 | 95.4 | Figure 3 |

### EXAMPLE 3

### Purification of montelukast acid by PEC

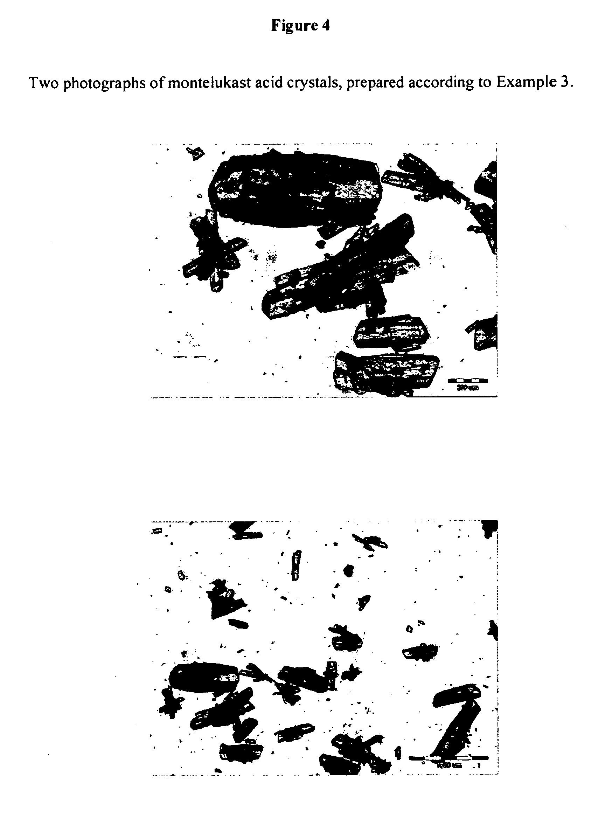

3.15 g of crude montelukast acid (purity 93.5%) with 0.15 % of the impurity 4, (R,E)-methyl 2-(1-((1-(3-(2-(7-chloroquinolin-2-yl)vinyl)phenyl)-3-(2-(2-hydroxypropan-2 yl)phenyl)propylthio)methyl)cyclopropyl)acetate, is completely dissolved in 9 mL of ethyl acetate at reflux conditions. 12 mL of warmed water and 0.35 g of Aerosol® OT-B (85 w/w % dioctyl sodium sulfosuccinate, 15 w/w % sodium benzoate) were added. Furthermore, the emulsion was cooled to 45 °C with average cooling ramp 0.5 K/min and after that the emulsion was slowly cooled with the average cooling ramp 0.10 K/min to 15 °C. The seeds of pure montelukast acid were added (50 mg) at 30 °C, during the slow cooling. Formed suspo-emulsion was homogenized at this temperature for the next 3 hours for montelukast acid to fully crystallize. The formed product is isolated by filtration and washed with 2 mL of water. 2.6 g of montelukast acid was obtained. The microscope pictures of formed product are presented in Figure 4.
Montelukast acid with 97.9 % purity and with 0.02 % of the impurity 4 was obtained.

The steps of the crystallization were repeated using the product from the Example 3 (97.9 % purity with 0.02 % of the impurity 4). 2.3 g of 99.3% pure montelukast acid with < 0.01 % of the impurity 4, was obtained.

**Table 3: HPLC analysis of crystallized materials in Example 3.**

| | ***Impurity 4 content (%)*** | ***Purity of crystallized material (%)*** | ***Microscopic pictures of formed particles*** |
|---|---|---|---|
| EXAMPLE 3 1^{st} crystallization | 0.02 | 97.2 | Figure 4 |
| EXAMPLE 3 2^{nd} crystallization | <0.01 | 99.3 | / |

### EXAMPLE 4

### Purification of orlistat by PEC

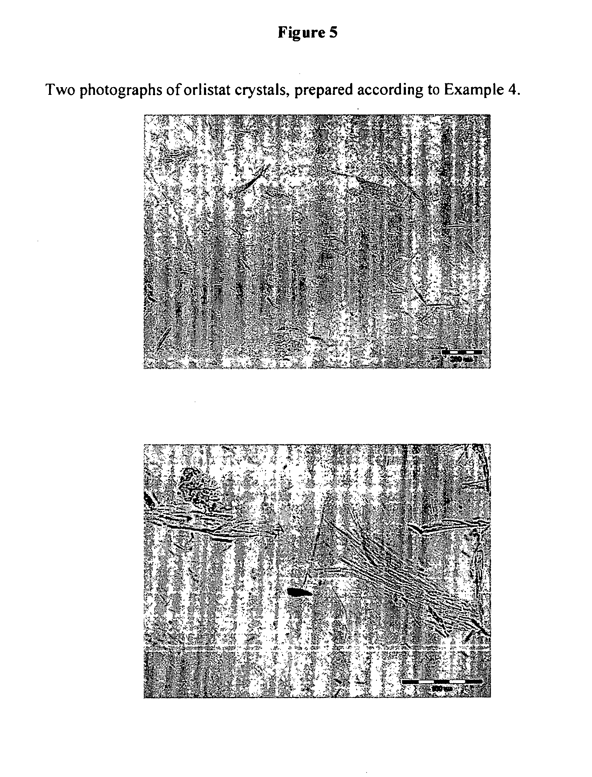

13 g of crude orlistat (purity 84.3 %, 83.2 w/w %) was added to an emulsion consisting of 55 mL of heptan, 23.5 mL of water and 0.4 g of Aerosol® OT-B (85 w/w dioctyl sodium sulfosuccinate, 15 w/w % sodium benzoate) and 1.5 g of Pluronic F=127 (poly(ethylene glycol)-block-poly(propylene glycol)-block-poly(ethylene glycol) tri-block co-polymer). The suspo-emulsion was heated from room temperature to 40 °C with average heating ramp I K/min and maintained at this temperature for 5 minutes for all solid to dissolve. Furthermore the emulsion was cooled to 21 °C with average cooling ramp 0.8 K/min and the seeds of pure orlistat were added (150 mg). Furthermore the temperature in reactor was cooled to 19 °C with average cooling ramp 0.02 K/min and maintained for the next 7 hours to allow orlistat to fully crystallize. The formed product was isolated by filtration and washed with 10 mL of water. 9.7 g of orlistat with purity of 97.2 % and 95.3 w/w % was isolated. The microscopic pictures of formed product are presented in Figure 5.

The process is repeated to obtain 8.6 g orlistat with 98.7 % purity.

### COMPARATIVE EXAMPLE 4

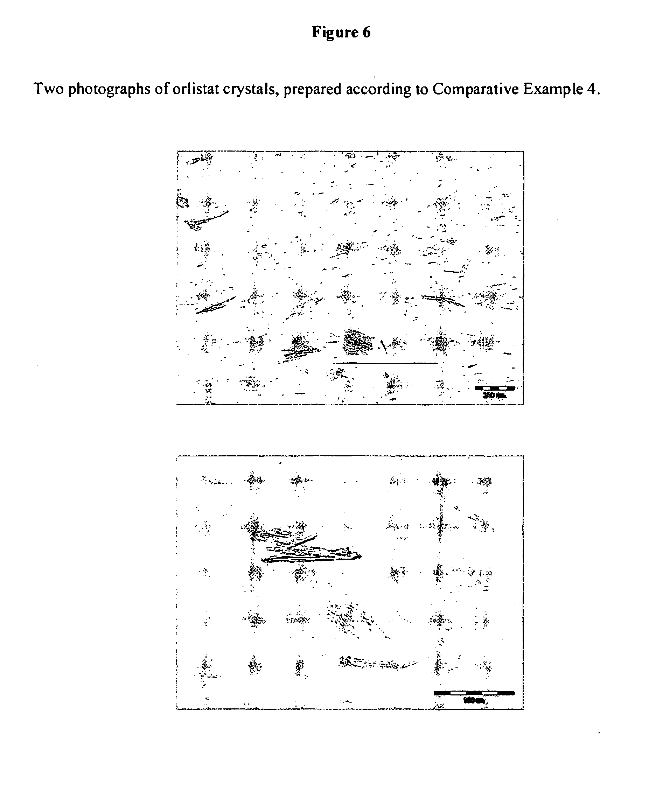

13 g of crude orlistat (purity 84.3 %, 83.2 w/w %) was added to an emulsion consisting of 55 mL of heptan, 23.5 mL of water and 0.4 g of Aerosol® OT-B (85 w/w % dioctyl sodium sulfosuccinate, 15 w/w % sodium benzoate) and 1.5 g of Pluronic® F-127 (poly(ethylene glycol)-block-poly(propylene glycol)-block-poly(ethylene glycol) tri-block co-polymer). The suspo-emulsion was heated from room temperature to 40 °C with average heating ramp I K/min and maintained at this temperature for 5 minutes for all solid to dissolve. Furthermore the emulsion was cooled to 19 °C with average cooling ramp 0.8 K/min and the seeds of pure orlistat were added (150 mg). The temperature was maintained for the next 8 hours to allow orlistat to fully crystallize. The formed product was isolated by filtration and washed with 10 mL of water. 9.6 g of orlistat with purity 94.7 % and 93.4 w/w % was obtained. The microscopic pictures of formed product are presented in Figure 6.

**Table 4: HPLC analysis of crystallized materials.**

| | ***Purity of crystallized material (%)*** | ***Microscopic pictures of formed particles*** |
|---|---|---|
| EXAMPLE 4 | 97.2 | Figure 5 |
| COMPARATIVE EXAMPLE 4 | 94.7 | Figure 6 |

### EXAMPLE 4/a

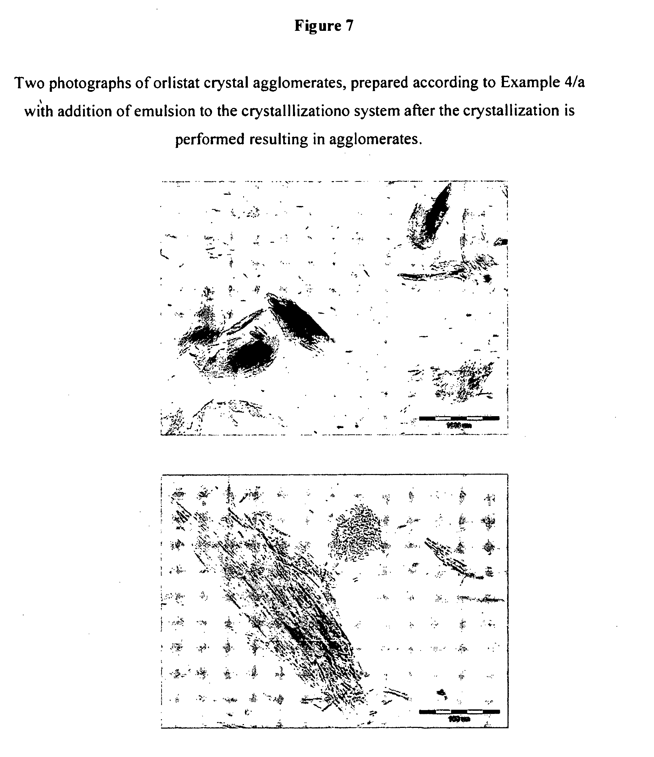

After the first crystallization was completed (Example 4, after the suspo-emulsion is maintained at 19 °C for 7 hours) emulsion consisting 20 mL of heptan, 10 mL of water and 0.8 g of Pluronic® F-127 (poly(ethylene glycol)-*block*-poly(propylene glycol)-*block-*poly(ethylene glycol) tri-block co-polymer) is added. The emulsion was prepared separately and homogenized for all F127 to dissolve before the addition to the suspo-emulsion. Finally, the suspo-emulsion was homogenized for the next 45 minutes and the product was isolated with filtration. 9.3 g of orlistat was isolated without affecting the purity of the formed product (comparable to Example 4).
The microscopic pictures of formed particles are presented in Figure 7.

### EXAMPLE 5

### Purification of atorvastatine intermediate by PEC

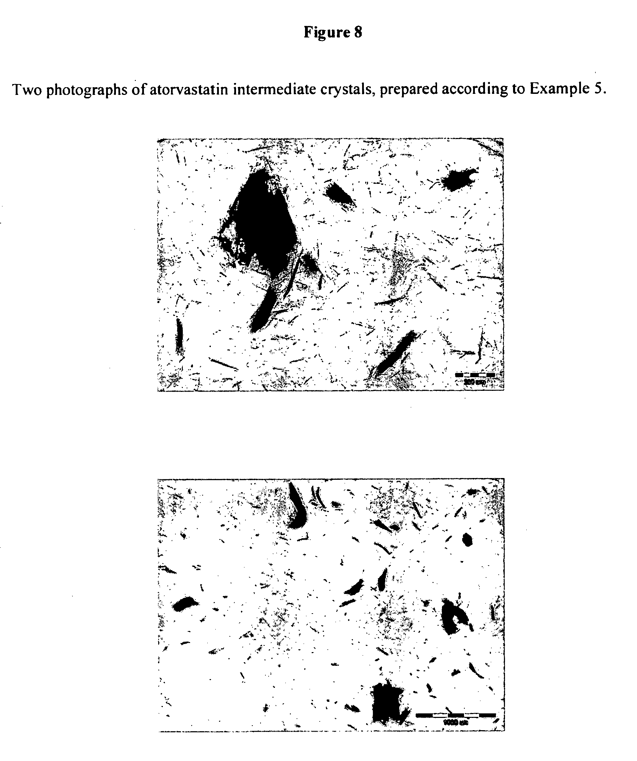

12g of crude atorvastatine intermediate (3R,5R)-tert-butyl 7-(2-(4-fluorophenyl)-5-isopropyl-3-phenyl-4-(phenylcarbamoyl)-1*H*-pyrrol-1-yl)-3,5-dihydroxyheptanoate (purity 98.8 %) with 0.22 % of the impurity 5 (MH+18, RRt = 1.03), was added to an emulsion consisting of 84 mL of ethyl acetate, 20 mL of water and 2.4 g of Aerosol® OT-B (85 w/w % dioctyl sodium sulfosuccinate, 15 w/w % sodium benzoate). The suspo-emulsion was heated from room temperature to 70 °C with average heating ramp 1 K/min and maintained at this temperature for 20 minutes for all solid to dissolve. Furthermore the emulsion was cooled to 15 °C with average cooling ramp 0.6 K/min and the seeds of pure product were added (200 mg). Furthermore the temperature in reactor was cooled to 8 °C with cooling ramp 0.04 K/min and maintained for the next 30 minutes to allow product to fully crystallize. Formed product was isolated by filtration and finally washed with 10 mL of water. 10.1 g of 99.4 % pure product with 0.14 % of the impurity 5 (MH+18, RRt = 1.03), was obtained.
The microscope pictures of formed product are presented in Figure 8.

**Table 5: HPLC analysis of crystallized atorvastatine intermediate.**

| | ***Impurity 5 content %, MH+18, RRt =* 1.03** | ***Purity of crystallized material (%)*** | ***Microscopic pictures of formed particles*** |
|---|---|---|---|
| EXAMPLE 5 | 0.14 | 99.4 | Figure 8 |

### EXAMPLE 6

### Purification of pantoprazole acid by PEC

An emulsion consisting 4 mL ethyl acetate, 4 mL of water, I g of Soprophor® FL and 0.4 g of sodium hydroxide was prepared. Furthermore 4 g of crude pantoprazole in acid form was added and heated up to 65 °C with average heating ramp 1K/min and maintained at this temperature for all solid to dissolve (10 minutes). The obtained emulsion was cooled to 20 °C with cooling ramp 0.5 K/min and stirred with mechanical stirrer. The seeds of pure pantoprazole acid were added (50 mg) and further cooled to 5 °C with average cooling ramp 0.5 K/min and maintained at this temperature for 15 hours. The crystallized pantoprazole acid was filtered and washed with 2 mL of butyl methyl ether and with 2 mL of water. The product was dried in a vacuum dryer at 40 °C for 10 hours. 3.2 g of product was obtained.

The purity of the starting crude pantoprazole and the pantoprazole purified by PEC were analysed by HPLC and are given in the table below:

**Table 6: HPLC analysis of crude pantoprazole acid and crystallized pantoprazole acid products.**

| **Component** | **Crude pantoprazole acid used** | **Crystallized pantoprazole acid** |
|---|---|---|
| | **purity (%)** | **purity (%)** |
| Pantoprazole acid | 94.70 | 99.65 |
| (Impurity 6) | 0.81 | 0.03 |
| 2-[[Chloro(3,4-dimethoxypyridin-2-yl)methyl]sulfinyl]-5-(difluoromethoxy)-1*H*-benzimidazole | | |
| (Impurity 7) | 1.97 | 0.25 |
| 2-[[Chloro(3,4-dimethoxypyridin-2-yl)methyl]sulfinyl]-6-(difluoromethoxy)-1*H*-benzimidazole | | |
| (Impurity 8) | 0.30 | 0.03 |
| 5-Difluoromethoxy-2-[[(3,4-dimethoxy-2-pyridinyl)methyl]thio]-1*H*-benzimidazole | | |
| (Impurity 9) | 0.16 | 0.02 |
| 6-(Difluoromethoxy)-2-((3,4-dimethoxypyridin-2-yl)methylsulfonyl)-1*H*-benzo[d]imidazole | | |

**Table 7: HPLC analysis of pantoprazole acid and the crystallized product after the crystallization (the steps of the described crystallization were preformed).**

| **Component** | **Pantoprazole used** | **Crystallized pantoprazole acid** |
|---|---|---|
| | **purity (%)** | **purity (%)** |
| Pantoprazole acid | 99.59 | 99.94 |
| (Impurity 6) | 0.03 | 0.00 |
| 2-[[Chloro(3,4-dimethoxypyridin-2-yl)methyl]sulfinyl]-5-(difluoromethoxy)-1*H*-benzimidazole | | |
| (Impurity 7) | 0.32 | 0.05 |
| 2-[[Chloro(3,4-dimethoxypyridin-2-yl)methyl]sulfinyl]-6-(difluoromethoxy)-1*H*-benzimidazole | | |
| (Impurity 8) | 0.02 | 0.01 |
| 5-Difluoromethoxy-2-[[(3,4-dimethoxy-2-pyridinyl)methyl]thio]-1*H*-benzimidazole | | |
| (Impurity 9) | 0.00 | 0.00 |
| 6-(Difluoromethoxy)-2-((3,4-dimethoxypyridin-2-yl)methylsulfonyl)-1*H*-benzo[d]imidazole | | |

### EXAMPLE 7

### Purification of candesartan intermediate by PEC

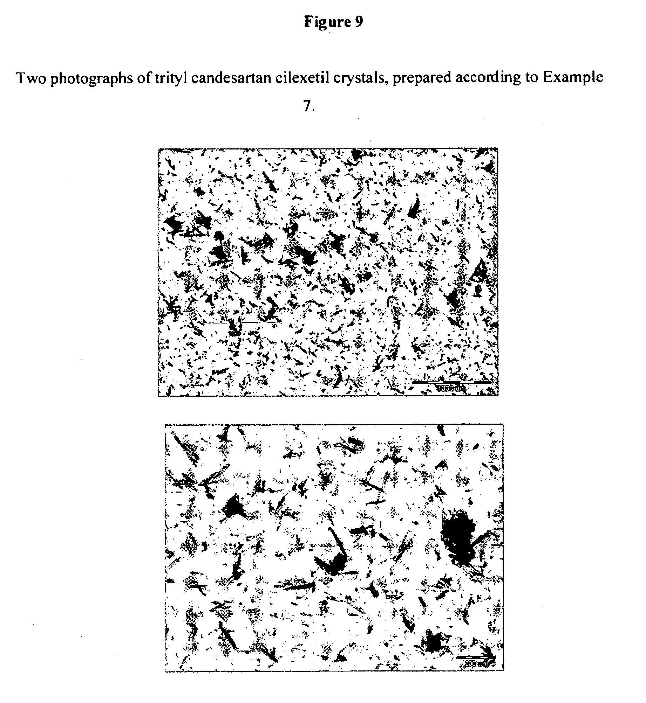

5 g of crude 1-(cyclohexyloxycarbonyloxy)ethyl 2-ethoxy-1-((2'-(1-trityl-1*H*-tetrazol-5-yl)biphenyl-4-yl)methyl)-1*H*-benzo[d]imidazole-7-carboxylate (95.9 w/w %, purity 99.0 %) with 0.68 % of the impurity 10, 2-ethoxy-1-((2'-(1-trityl-1*H*-tetrazol-5-yl)biphenyl-4-yl)methyl)-1H-benzo[d]imidazole-7-carboxylic acid, and 0.04 % of the impurity 11, ethyl 2-ethoxy-1-((2'-(1-trityl-1*H*-tetrazol-5-yl)biphenyl-4-yl)methyl)-1*H*-benzo[d]imidazole-7-carboxylate, was added to 8.7 mL of dichloromethane and heated to reflux for all solid to dissolve (solution A). Separately the mixture of 23 mL of water, 28 mL of acetonitrile and 0.85 g of Aerosol® OT-B (85 w/w % dioctyl sodium sulfosuccinate, 15 w/w % sodium benzoate) was prepared and heated to 60 °C (mixture B). Then the mixture B was added to the solution A. The formed emulsion was cooled to 30 °C with the average cooling ramp 0.6 K/min and the seed crystals of pure trityl candesartan cilexetil were added (150 mg). Furthermore the temperature in the reactor was slowly cooled to 20 °C with an average cooling ramp 0.05 K/min and maintained for the next 15 hours to allow the product to fully crystallize. The formed product is isolated by filtration and washed with 10 mL of acetonitrile. 4.5 g of the product with purity of 99.6 %, with 0.24 % of the impurity 10 and 0.02 % of the impurity 11, was isolated.
The microscope pictures of formed product are presented in Figure 9.

### COMPARATIVE EXAMPLE 7

### Purification of candesartan intermediate by PEC

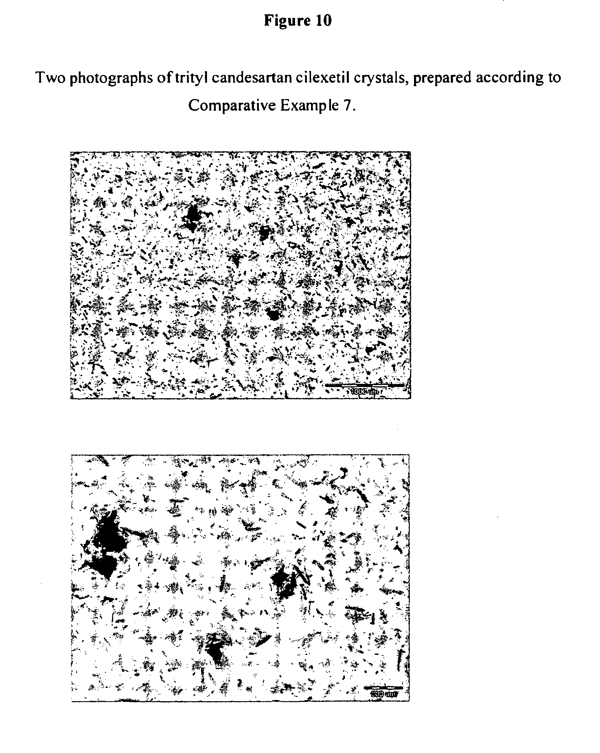

5 g of crude 1-(cyclohexyloxycarbonyloxy)ethyl 2-ethoxy-1-((2'-(1-trityl-1*H*-tetrazol-5-yl)biphenyl-4-yl)methyl)-1*H*-benzo[*d*]imidazole-7-carboxylate (95.9 w/w %, purity 99.0 %) with 0.68 % of the impurity 10, 2-ethoxy-1-((2'-(1-trityl-1*H*-tetrazol-5-yl)biphenyl-4-yl)methyl)-1*H*-benzo[d]imidazole-7-carboxylic acid, and 0.04 % of the impurity 11, ethyl 2-ethoxy-1-((2'-(1-trityl-1*H*-tetrazol-5-yl)biphenyl-4-yl)methyl)-1*H*-benzo[*d*]imidazole-7-carboxylate, was added to 8.7 mL of dichloromethane and heated to reflux for all solid to dissolve (solution A). Separately the mixture of 23 mL of water, 28 mL of acetonitrile and 0.85 g of Aerosol® OT-B (85% w/w% dioctyl sodium sulfosuccinate, 15 w/w % sodium benzoate), was prepared and heated to 60 °C (mixture B). Then the mixture B was added to the solution A. The formed emulsion was cooled to 30 °C with average cooling ramp 0.6 K/min and the seed crystals of pure trityl candesartan cilexetil were added (150 mg). Furthermore the temperature in reactor was slowly cooled to 20 °C with average cooling ramp 0.05 K/min and maintained at the final temperature for the next 15 hours to allow the product to fully crystallize. Formed product is isolated by filtration and washed with 10 mL of acetonitrile. 4.3 g of the product with purity of 99.5 %, with 0.27 % of the impurity 10 and 0.02 % of the impurity 11, was isolated
The microscope pictures of formed product are presented in Figure 10.

**Table 9: HPLC analysis of crystallized materials.**

| | ***Impurity 10 content (%)*** | ***Impurity 11 content (%)*** | ***Purity of crystallized material (%)*** | ***Microscopic pictures of formed particles*** | ***Particle size distribution curve*** |
|---|---|---|---|---|---|
| EXAMPLE 7 | 0.24 | 0.02 | 99.6 | Figure 13 | Figure 16 |
| COMPARATIVE EXAMPLE 7 | 0.27 | 0.02 | 99.5 | Figure 14 | Figure 16 |

### EXAMPLE 8

### Purification of candesartan intermediate by PEC

24.0 g of crude 1-(cyclohexyloxycarbonyloxy)ethyl 2-ethoxy-1-((2'-(1-trityl-1*H*-tetrazol-5-yl)biphenyl-4-yl)methyl)-1*H*-benzo[*d*]imidazole-7-carboxylate (50.0 w/w %, purity 68.3 %) with 3.55 % of the impurity 10, 2-ethoxy-1-((2'-(1-trityl-1*H*-tetrazol-5-yl)biphenyl-4-yl)methyl)-1*H*-benzo[d]imidazole-7-carboxylic acid, and 0.20 % of the impurity 11, ethyl 2-ethoxy-1-((2'-(1-trityl-1*H*-tetrazol-5-yl)biphenyl-4-yl)methyl)-1*H-*benzo[*d*]imidazole-7-carboxylate, was added to 11 mL of dichloromethane and heated to reflux for all solid to dissolve (solution A). Separately the mixture of 43.6 mL of water, 83.7 mL of acetonitrile and 3.2 g of Aerosol® OT-B (85 w/w % dioctyl sodium sulfosuccinate, 15 w/w % sodium benzoate) was prepared and heated to 60 °C (mixture B). Then the mixture B was added to the solution A. The formed emulsion was cooled to 30 °C with average cooling ramp 0.6 K/min and the seed crystals of pure trityl candesartan cilexetil were added (350 mg). Furthermore the temperature in reactor was slowly cooled to 15 °C with average cooling ramp 0.05 K/min and maintained for the next 24 hours to allow the product to fully crystallize. Formed product is isolated by filtration and washed with 10 mL of acetonitrile. 10.9 g of the product with purity of 96.0 %, 94.0 w/w %, with 1.80 % of the impurity 10 and 0.08 % of the impurity 11, was isolated.

### EXAMPLE 9

### Purification of candesartan intermediate by PEC

7.0 g of crystallized 1-(cyclohexyloxycarbonyloxy)ethyl 2-ethoxy-1-((2'-(1-trityl-1*H-*tetrazol-5-yl)biphenyl-4-yl)methyl)-1*H*-benzo[d]imidazole-7-carboxylate, the product from Example 8 (purity 96.0 %, 94.0 w/w %) with 1.80 % of the impurity 10, 2-ethoxy-1-((2'-(1-trityl-1*H*-tetrazol-5-yl)biphenyl-4-yl)methyl)-1*H*-benzo[d]imidazole-7-carboxylic acid, and 0.08 % of the impurity 11, ethyl 2-ethoxy-1-((2'-(1-trityl-1*H-*tetrazol-5-yl)biphenyl-4-yl)methyl)-1*H*-benzo[d]imidazole-7-carboxylate, was added to 12.1 mL of dichloromethane and heated to reflux for all solid to dissolve (solution A). Separately the mixture of 32.6 mL of water, 37 mL of acetonitrile and 1.4 g of Aerosol® OT-B (85 w/w % dioctyl sodium sulfosuccinate, 15 w/w % sodium benzoate) was prepared and heated to 60 °C (mixture B). Then the mixture B was added to the solution A. The formed emulsion was cooled to 30 °C with average cooling ramp 0.6 K/min and the seed crystals of pure trityl candesartan cilexetil were added (200 mg). Furthermore the temperature in reactor was slowly cooled to 25 °C with average cooling ramp 0.05 K/min and maintained for the next 12 hours to allow the product to fully crystallize. Formed product is isolated by filtration and washed with 10 mL of acetonitrile. 6.0 g of the product with purity of 99.3 % (> 98.0 w/w %), with 0.45 % of the impurity 10 and 0,00 % of the impurity 11, was isolated.

### EXAMPLE 10

### Purification of orlistat by PEC

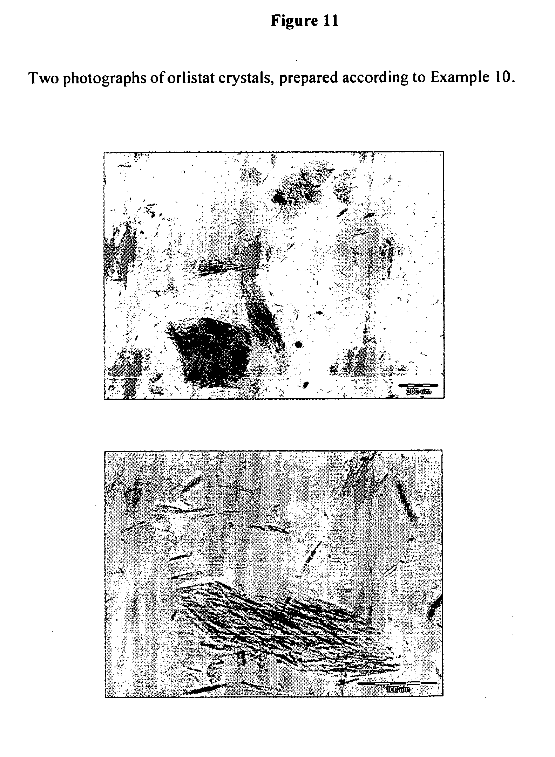

10 g of crude orlistat (purity 95.3 %, 94.8 w/w %) with 0.37 % of the impurity 12, (S)-(3S,4R,6S)-3-hexyl-2-oxo-6-undecyltetrahydro-2*H*-pyran-4-yl 2-formamido-4-methylpentanoate, was added to an emulsion consisting of 39.5 mL of heptan, 17 mL of water and 0.57 g of Aerosol® OT-B (85 w/w % sodium dioctyl sulfosuccinate, 15 w/w % sodium benzoate). The suspo-emulsion was heated from room temperature to 40 °C with average heating ramp 1 K/min and maintained at this temperature for 5 minutes for all solid to dissolve. Furthermore the emulsion was cooled to 20 °C with average cooling ramp I K/min and the seeds of pure orlistat were added (200 mg). Furthermore the temperature in reactor was cooled to 18 °C with average cooling ramp 0.01 K/min and maintained for the next 7 hours to allow orlistat to fully crystallize. The formed product was isolated by filtration and washed with 15 mL of water. 8.4 g of orlistat with 98.5 % purity and 98.5 w/w %, with 0.18 % of the impurity 12, was isolated.
The microscopic pictures of formed product are presented in Figure 11.

### COMPARATIVE EXAMPLE 10

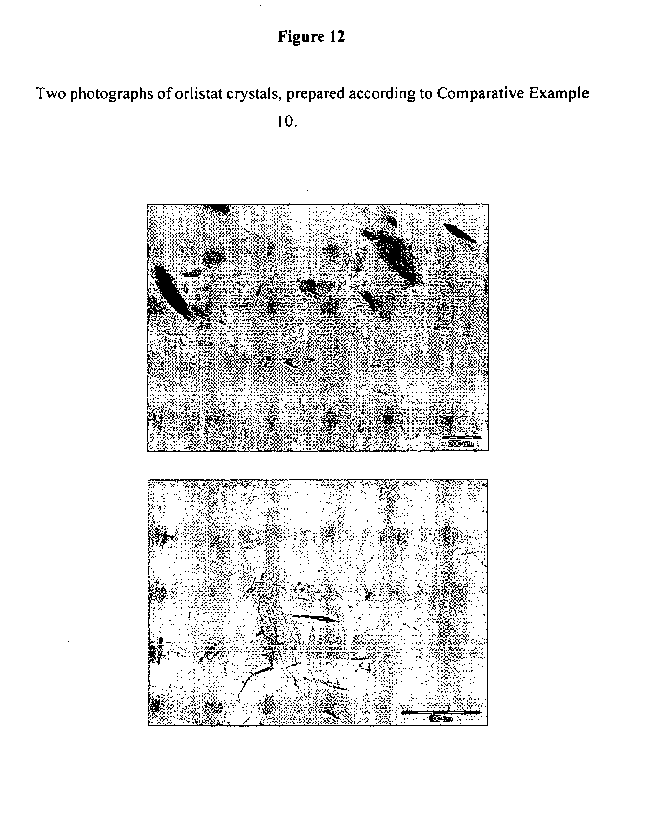

10 g of crude orlistat (purity 95.3 %, 94.8 w/w %) with 0.37 % of the impurity 12, (S)-(3S,4R,6S)-3-hexyl-2-oxo-6-undecyltetrahydro-2*H*-pyran-4-yl 2-formamido-4-methylpentanoate, was added to an emulsion consisting of 39.5 mL of heptan, 17 mL of water and 0.57 g of Aerosol® OT-B (85 w/w % sodium dioctyl sulfosuccinate, 15 w/w % sodium benzoate). The suspo-emulsion was heated from room temperature to 40 °C with average heating ramp I K/min and maintained at this temperature for 5 minutes for all solid to dissolve. Furthermore the emulsion was cooled to 18 °C with average cooling ramp I K/min and the seeds of pure orlistat were added (200 mg). The suspo-emulsion was maintained at the temperature 18 °C for the next 10 hours to allow orlistat to fully crystallize. The formed product was isolated by filtration and washed with 15 mL of water. 8.4 g of orlistat with 98.3 % purity and 97.5 w/w %, with 0.25 % of the impurity 12, was isolated. The microscopic pictures of formed product are presented in Figure 12.

**Table 10: HPLC analysis of crystallized materials after the first crystallization.**

| | ***Impunity 12 content (%)*** | ***Purity of crystallized material (%)*** | ***Microscopic pictures of formed particles*** | ***Particle size distribution curve*** |
|---|---|---|---|---|
| EXAMPLE 10 | 0.18 | 98.5 | Figure 11 | Figure 17 |
| COMPARATIVE EXAMPLE 10 | 0.25 | 98.3 | Figure 12 | Figure 17 |

### EXAMPLE 11

### Purification of montelukast acid by PEC

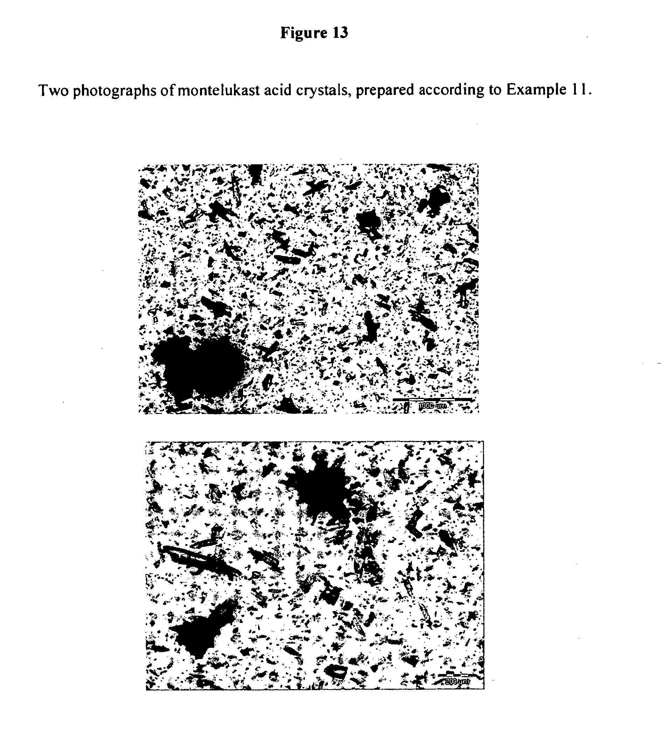

8.7 g of crude montelukast acid (purity 89.1 %, 87.0 w/w %) with 0.32 % of the impurity 13, (R,E)-2-(1-((3-(2-acetylphenyl)-1-(3-(2-(7-chloroquinolin-2-yl)vinyl)phenyl)propylthio) methyl) cyclopropyl)acetic acid, is completely dissolved in 18 mL of ethyl acetate at reflux conditions. 26 mL of water and 1.0 g of Aerosol® OT-B (85% w/w % dioctyl sodium sulfosuccinate, 15% w/w % sodium benzoate) were added. Furthermore, the emulsion was cooled to 50 °C and seeded with crystals of pure montelukast acid (150 mg). After that the emulsion was slowly cooled with the average cooling ramp 0.05 K/min to 10 °C. Formed suspo-emulsion was homogenized at the final temperature 10 °C for the next 5 hours for montelukast acid to fully crystallize. The formed product is isolated with filtration and washed with 6 mL of water.
6.4 g of montelukast acid (purity 96.2 %, 95.3 w/w %), with 0.21 % of the impurity 13, was obtained.
The microscope pictures of formed product are presented in Figure 13.

### COMPARATIVE EXAMPLE 11

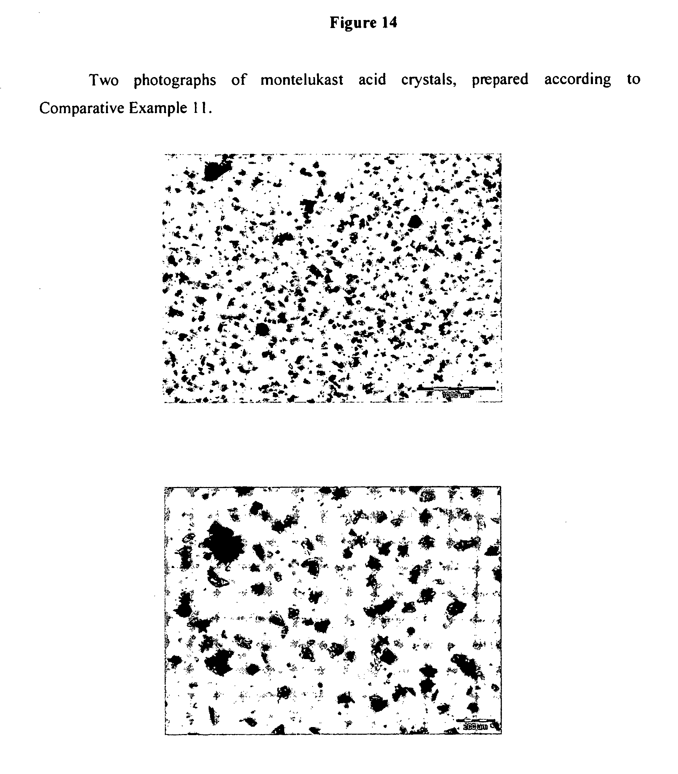

8.7 g of crude montelukast acid (purity 89.1 %, 87.0 w/w %) with 0.32 % of the impurity 13, (R,E)-2-(1-((3-(2-acetylphenyl)-1-(3-(2-(7-chloroquinolin-2-yl)vinyl)phenyl)propylthio) methyl) cyclopropyl)acetic acid, is completely dissolved in 18 mL of ethyl acetate at reflux conditions. 26 mL of water and 1.0 g of Aerosol® OT-B (85% w/w % dioctyl sodium sulfosuccinate, 15% w/w % sodium benzoate) were added. Furthermore, the emulsion was cooled to 10 °C and seeded with crystals of pure montelukast acid (150 mg). Formed suspo-emulsion was homogenized at the temperature 10 °C for the next 17 hours for montelukast acid to fully crystallize. The formed product is isolated with filtration and washed with 6 mL of water.
6.2 g of montelukast acid (purity 95.7 %, 94.1 w/w %), with 0.26 % of the impurity 13, was obtained.
The microscope pictures of formed product are presented in Figure 14.

**Table 8: HPLC analysis of crystallized materials.**

| ***Impurity*** | ***Impurity 13 content (%)*** | ***Purity of crystallized material (%)*** | ***Microscopic pictures of formed particles*** | ***Particle size distribution curve*** |
|---|---|---|---|---|
| EXAMPLE II | 0.21 | 96.2 | Figure 13 | Figure 15 |
| COMPARATIVE EXAMPLE II | 0.26 | 95.7 | Figure 14 | Figure 15 |

### EXAMPLE 12

### Purification of montelukast acid by PEC

i.) 1.9 g of crystallized montelukast acid - product from Example I (purity 96.2 %; 95.3 w/w %) with 0.21 % of the impurity 13, (R,E)-2-(1-((3-(2-acetylphenyl)-1-(3-(2-(7-chloroquinolin-2-yl)vinyl)phenyl)propylthio)methyl)cyclopropyl)acetic acid, was completely dissolved in 4.5 mL of ethyl acetate at reflux conditions. 6.5 mL of water and 0.25 g of Aerosol® OT-B (85% w/w dioctyl sodium sulfosuccinate, 15% w/w sodium benzoate) were added. Furthermore, the emulsion was cooled to 50 °C and seeded with crystals of pure montelukast acid (40 mg). After that the emulsion was slowly cooled with the average cooling ramp 0.05 K/min to 10 °C. Formed suspo-emulsion was homogenized at the final temperature 10 °C for the next 5 hours for montelukast acid to fully crystallize. The formed product is isolated with filtration and washed with 2 mL of water.
   1.6 g of montelukast acid (purity 97.8 %, 98.3 w/w %), with 0.15 % of the impurity 13, was obtained.
ii.) 1 g of crystallized montelukast acid - product from Example 12, i (purity 97.8 %, 98.3 w/w %) with 0.15 % of the impurity 13, (R,E)-2-(1-((3-(2-acetylphenyl)-1-(3-(2-(7-chloroquinolin-2-yl)vinyl)phenyl)propylthio)methyl)cyclopropyl)acetic acid, was completely dissolved in 4.5 mL of ethyl acetate at reflux conditions. 3.6 mL of water and 0.14 g of Aerosol® OT-B (85% w/w dioctyl sodium sulfosuccinate, 15% w/w sodium benzoate) were added. Furthermore, the emulsion was cooled to 50 °C and seeded with crystals of pure montelukast acid (40 mg). After that the emulsion was slowly cooled with the average cooling ramp 0.05 K/min to 10 °C. Formed suspo-emulsion was homogenized at the final temperature 10 °C for the next 5 hours for montelukast acid to fully crystallize. The formed product is isolated with filtration and washed with 2 mL of water.
   0.8 g of montelukast acid (purity 99.0 %, 98.8 w/w %), with 0.09 % of the impurity 13, was obtained.

### EXAMPLE 13

### Purification of montelukast acid by crystallization of 1-[[[[3-[(1E)-2-(7-chloroquinoline-2-yl)ethenyl]phenyl]-3-[2-(1-hydroxy-1-methylethyl)phenyl]propyl]thio]methyl] cyclopropane acetic acid trans-2,5-dimethylpiperazine salt

To a 50 mL round-bottom flask equipped with a magnetic mixer, a reflux condenser and a nitrogen inlet 4.8 g of crude montelukast acid (purity 76.4 %, 62 w/w %), 33 mL of ethyl acetate and 0.30 g of trans-2,5-dimethylpiperazine were added. The mixture was heated to 70 °C and a clear solution was obtained. Then the mixture was cooled to 20 °C and homogenized at this temperature for the next 15 hours.
The obtained product was isolated using filtration and washed with 50 mL of ethyl acetate/TBME mixture (50 v/v %).
2.70 g of montelukast-trans-2,5-dimethylpiperazine salt was obtained (purity 95.0 %).

2 g of obtained montelukast trans-2,5-dimethylpiperazine salt, 20 mL of ethylacetate and 20 mL of water was charged into a 50 mL round-bottom flask equipped with a magnetic mixer, a reflux condenser, a nitrogen inlet and an addition funnel. The mixture was heated to 70 °C for solid particles to dissolve. The formed emulsion was cooled to 20 °C and 1.6 mL solution of acetic acid in water (2M solution) was added drop wise. The mixture was stirred for the next 10 minutes. The organic phase was separated and washed twice with 20 mL of water at room temperature. Finally, the ethyl acetate was evaporated and 1.7 g of montelukast free acid was isolated (purity 94.4 %, 93 w/w %).

Montelukast free acid as obtained by the process above may further be purified by the process of the present invention. Alternatively montelukast salts can also be purified by the process of the present invention. As another possibility montelukast acid is purified by the emulsion crystallization process of the present invention without previous formation of a montelukast salt, such as montelukast piperazine salts.

### EXAMPLE 14

### Purification of montelukast acid by crystallization of 1-[[[[3-[(1E)-2-(7-chloroquinoline-2-yl)ethenyl]phenyl]-3-[2-(1-hydroxy-1-methylethyl)phenyl]propyl]thio]methyl]cyclo propane acetic acid trans-2,5-dimethylpiperazine salt

To a 50 mL round-bottom flask equipped with a magnetic mixer, a reflux condenser and a nitrogen inlet 4.8 g of crude montelukast acid (purity 76.4 %, 62 w/w %), 33 mL of ethyl acetate and 0.33 g of trans-2,5-dimethylpiperazine were added. The mixture was heated to 70 °C and a clear solution was obtained. Then the mixture was cooled to 20 °C and homogenized at this temperature for the next 15 hours.
The obtained product was isolated using filtration and washed with 50 mL of ethyl acetate/TBME mixture (50 v/v %).
2.75 g of montelukast-trans-2,5-dimethylpiperazine salt was obtained (purity 95.7 %).

2 g of obtained montelukast-trans-2,5-dimethylpiperazine salt, 20 mL of ethylacetate and 20 mL of water was charged into a 50 mL round-bottom flask equipped with a magnetic mixer, a reflux condenser, a nitrogen inlet and an addition funnel. The mixture was heated to 70 °C for solid particles to dissolve. The formed emulsion was cooled to 20 °C and 2.8 mL solution of acetic acid in water (2M solution) was added drop wise. The mixture was stirred for the next 10 minutes. The organic phase was separated and washed twice with 20 mL of water at room temperature. Finally, the ethyl acetate phase was evaporated and 1.7 g of montelukast free acid was isolated (purity 95.0 %, 92.7 w/w %).

Montelukast free acid as obtained by the process above may further be purified by the process of the present invention. Alternatively montelukast salts can also be purified by the process of the present invention. As another possibility montelukast acid is purified by the emulsion crystallization process of the present invention without previous formation of a montelukast salt, such as montelukast piperazine salts.

### EXAMPLE 15

### Purification of montelukast acid by crystallization of 1-[[[[3-[(1E)-2-(7-chloroquinoline-2-yl)ethenyl]phenyl]-3-[2-(1-hydroxy-1-methylethyl)phenyl]propyl]thio]methyl] cyclopropane acetic acid 2-methylpiperazine salt

To a 50 mL round-bottom flask equipped with a magnetic mixer, a reflux condenser and a nitrogen inlet 3 g of crude montelukast acid (purity 78.3 %, w/w %), 32 mL of ethyl acetate and 0.27 g 2-methylpiperazine were added. The mixture was heated to 70 °C and a clear solution was obtained. Then the mixture was cooled to 20 °C and homogenized at this temperature for the next 15 hours. The obtained product was isolated using filtration and washed with 50 mL of ethyl acetate/TBME mixture (50 v/v %). 1.60 g of dry montelukast-2-methylpiperazine salt was obtained (purity 97.9 %).

I g of obtained montelukast-2-methylpiperazine salt, 10 mL of ethylacetate and 10 mL of water was charged into a 50 mL round-bottom flask equipped with a magnetic mixer, a reflux condenser, a nitrogen inlet and an addition funnel. The mixture was heated to 70 °C for solid particles to dissolve. The formed emulsion was cooled to 20 °C and 1.4 mL solution of acetic acid in water (2M solution) was added drop wise. The mixture was stirred for the next 10 minutes and then stopped. The organic phase was separated and washed twice with 10 mL of water at room temperature. Finally, the ethyl acetate phase was evaporated and 0.8 g of montelukast free acid was isolated (purity 98.0 %, 97.8 w/w %).

Montelukast free acid as obtained by the process above may further be purified by the process of the present invention. Alternatively montelukast salts can also be purified by the process of the present invention. As another possibility montelukast acid is purified by the emulsion crystallization process of the present invention without previous formation of a montelukast salt, such as montelukast piperazine salts.

### Structures of molecules purified and related impurities:

### -Ezetimibe (Example 1)

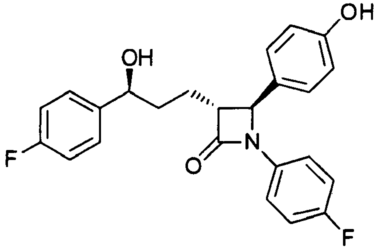

(3R,4S)-1-(4Fluorophenyl)-3-[(S)-3-(4-fluorophenyl)-3-hydroxypropyl]-4-(4-hydroxyphenyl)azetidin-2-one

### -Impurity 1 (Example 1)

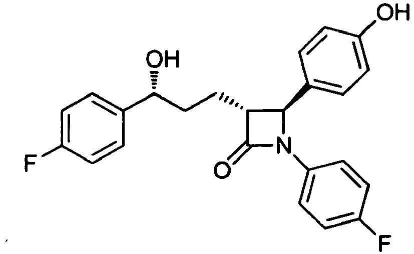

(3R,4S)-1-(4-Fluorophenyl)-3-[(R)-3-(4-fluorophenyl)-3-hydroxypropyl]-4-(4-hydroxyphenyl)azetidin-2-one

### - Fipronil (Example 2, Comparative Example 2)

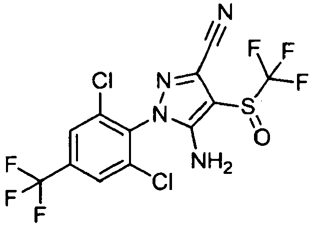

5-Amino-1-[2,6-dichloro-4-(trifluoromethyl)phenyl]-4-(trifluoromethylsulfinyl)-1 H-pyrazole-3-carbonitrile

### -Impurity 2 (Example 2, Comparative Example 2)

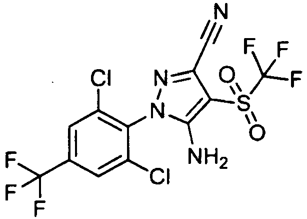

5-Amino-1-(2,6-dichloro-4-(trifluoromethyl)phenyl)-4-(trifluoromethylsulfonyl)-1*H-*pyrazole-3-carbonitrile

### -Impurity 3 (Example 2, Comparative Example 2)

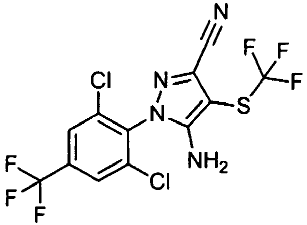

5-Amino-1-(2,6-dichloro-4-(trifluoromethyl)phenyl)4-(trifluoromethylthio)-1*H-*pyrazole-3-carbonitrile

### -Montelukast acid (Example 3)

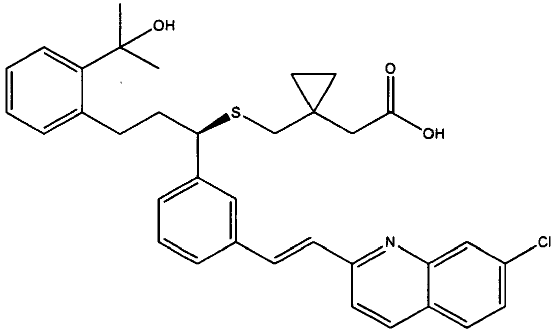

1-[[[(1R)-1-[3-[(1E)-2-(7-Chloro-2-quinolinyl)ethenyl]phenyl]-3-[2-(1-hydroxy-1-methylethyl)phenyl]propyl]thio]methyl]cyclopropaneacetic acid

### -Impurity 4 (Example 3)

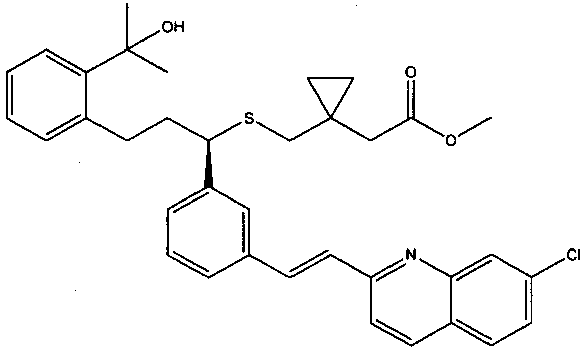

Methyl 1-[[[(1R)-1-[3-[(1E)-2-(7-Chloro-2-quinolinyl)ethenyl]phenyl]-3-[2-(1-hydroxy-1-methylethyl)phenyl]propyl]thio]methyl]cyclopropaneacetate

### -Orlistat (Example 4, Comparative Example 4)

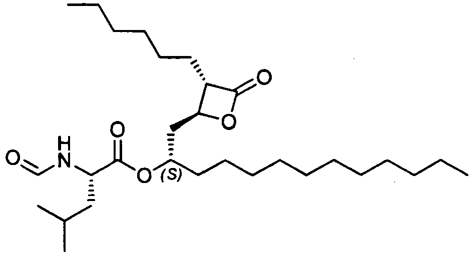

### N-Formyl-L-leucine (1S)-1-[[(2S,3S)-3-hexyl-4-oxo-2-oxetanyl]methyl]dodecyl ester

### -Atorvastatin intermediate (Example 5)

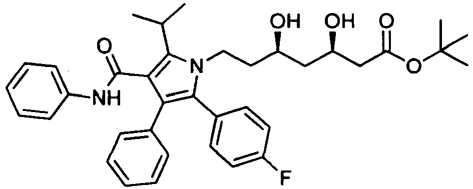

(3R,5R)-*Tert*-butyl 7-(2-(4-fluorophenyl)-5-isopropyl-3-phenyl-4-(phenylcarbamoyl)-1*H*-pyrrol-1-yl)-3,5-dihydroxyheptanoate

### -Impurity 5 (Example 5)

(3R,5R)-*Tert*-butyl 7-(2-(4-fluorophenyl)-5-isopropyl-3-phenyl-4-(phenylcarbamoyl)-1*H*-pyrrol-1-yl)-3,5-dihydroxyheptanoate MH+18 (RRt = 1.03)

### -Pantoprazole acid (Example 6)

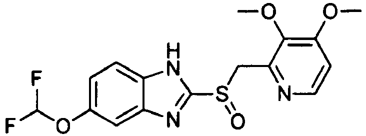

5-(Difluoromethoxy)-2-[[(3,4-dimethoxy-2-pyridinyl)methyl]sulfinyl]-1*H*-benzimidazole

### -Impurity 6 (Example 6)

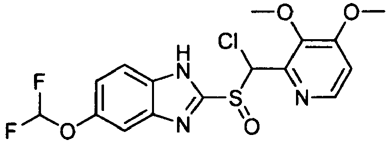

2-[[Chloro(3,4-dimethoxypyridin-2-yl)methyl]sulfinyl]-5-(difluoromethoxy)-1*H*-benzimidazole

### -Impurity 7 (Example 6)

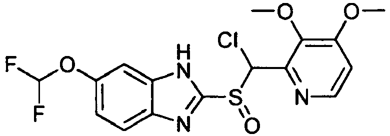

2-[[Chloro(3,4-dimethoxypyridin-2-yl)methyl]sulfinyl]-6-(difluoromethoxy)-1*H*-benzimidazole

### -Impurity 8 (Example 6)

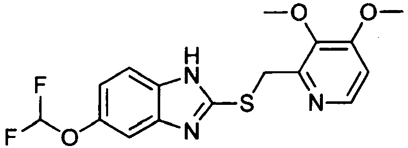

5-Difluoromethoxy-2-[[(3,4-dimethoxy-2-pyridinyl)methyl]thio]-1*H*-benzimidazole

### -Impurity 9 (Example 6)

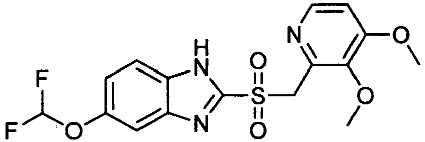

5-(Difluoromethoxy)-2-[[(3,4-dimethoxypyridin-2-yl)methyl]sulfonyl]-1*H*-benzimidazole

### -Trityl Candesartan cilexetil (Example 7)

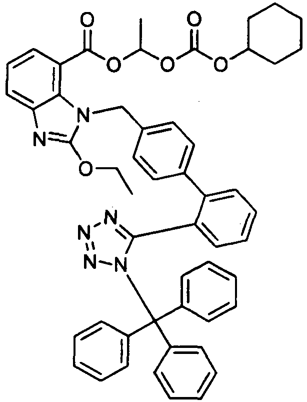

1-[[(Cyclohexyloxy)carbonyl]oxy]ethyl 2-ethoxy-1-[[2'-(1-trityl-1*H*-tetrazol-5-yl)-[1,1'-biphenyl]-4-yl]methyl]-1*H*-benzimidazole-7-carboxylate

### -Impurity 10 (Example 7)

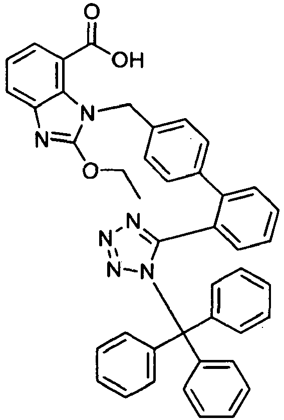

2-Ethoxy-1-((2'-(1-trityl-1*H*-tetrazol-5-yl)-[1,1'-biphenyl]-4-yl)methyl)-1*H-*benzo[d]imidazole-7-carboxylic acid

### -Impurity 11 (Example 7)

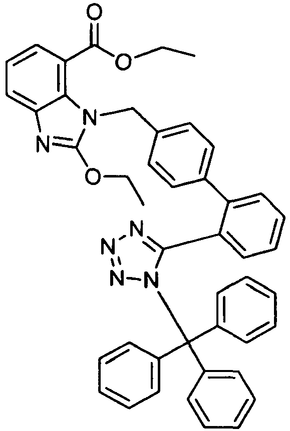

Ethyl 2-ethoxy-1-((2'-(1-trityl-1*H*-tetrazol-5-yl)-[1,1'-biphenyl]-4-yl)methyl)-1 H-benzo[d]imidazole-7-carboxylate

### - Impurity 12 (Example 15)

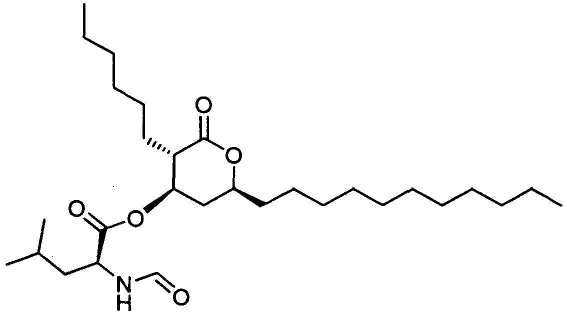

(*S*)-(3*S*,4*R*,6*S*)-3-Hexyl-2-oxo-6-undecyltetrahydro-2*H*-pyran-4-yl 2-formamido-4-methylpentanoate

### -Impurity 13

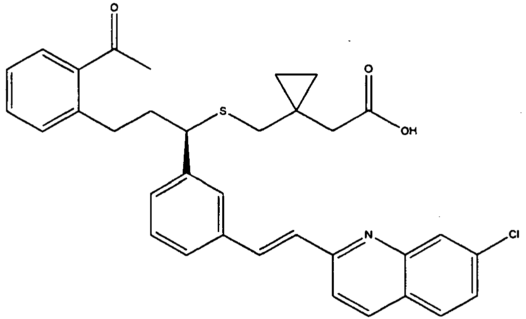

(R,E)-2-(1-((3-(2-Acetylphenyl)-1-(3-(2-(7-chloroquinolin-2-yl)vinyl)phenyl)propylthio)methyl)cyclopropyl)acetic acid

## Claims

1. A crystallization process for the preparation of chemical compounds, in particular of pure chemical compounds, in which an emulsion of droplets in a continuous phase is formed having Gibbs free enthalpy of formation ΔG>0, to effect crystallization and purification of the crude substance in the continuous or droplet phase, said emulsion comprising a surface active agent, **characterized in that** seeding with crystals of the pure substance is carried out at a higher temperature than the temperature at which the crystallization is completed.

2. The process according to claim 1, **characterized in that** the purity of the crude substance is less than 90 %(w/w), preferably less than 80 %(w/w), most preferably less than 60% (w/w).

3. The process according to any of claims 1 or 2, where the final targeted temperature, at which the crystallization is completed, is 2-50 °C below the temperature of seeding.

4. The process according to any of claims 1 to 3, where the temperature of seeding is 0-65 °C lower, preferably 0.5-65 °C lower, preferably 2-65 °C lower, than the temperature, at which all of aggregate mixture is dissolved.

5. The process according to any of claims 1 to 4, where the average cooling rate for cooling the crystallization mixture from temperature, at which the seeding is done, to the targeted temperature is below 1 K/min, preferably below 0.5 K/min.

6. The process according to any of claims 1 to 5, where emulsion comprises an C₁-C₅ acetate ester as an organic phase.

7. The process according to any of claims 1 to 6 **characterized in that** agglomeration of formed particles is induced by addition of an additional amount of the emulsion to the suspo-emulsion present after seeding and/or during crystallisation.

8. The process according to any of claims 1 to 7 used for the purification of crude substance, said crude substance comprising one selected from the group consisting of statins like rosuvastatin, fluvastatin, simvastatin, lovastatin, atorvastatin, and their intermediates, like (3R,5R)-tert-butyl 7-(2-(4-fluorophenyl)-5-isopropyl-3-phenyl-4-(phenylcarbamoyl)-1*H*-pyrrol-1-yl)-3,5-dihydroxyheptanoate; sartans like valsartan, losartan, candesartan cilexetyl, olmesartan, telmisartan and irbesartan or their intermediates like trityl candesartan cilexetyl; prazoles like lansoprazole, pantoprazole, rabeprazole, omeprazole, or esomeprazole, ezetimibe, fipronil, montelukast acid or montelukast salts, like montelukast 2-methylpiperazine salt or 2-methylpiperazine salt; orlistat or its intermediate lipstatin, solifenacin base or solifenacin salts, and quetiapine; preferably one selected from the group consisting of orlistat, trityl candesartan cilexetyl and montelukast acid.

9. The process according to any of claims 1 to 8 used for the purification of crude substance, said crude substance comprising one selected from the group consisting of candesartan cilexetyl, trityl candesartan cilexetyl and any intermediate of candesartan, with a purity of more than 95 % (w/w) in a single crystallization step and a resulting purity of more than 99 % (w/w) after two crystallization steps.

10. The process according to claim 9 where the purity of the crude substance comprising one selected from the group consisting of candesartan cilexetyl, trityl candesartan cilexetyl and any intermediate of candesartan is below 90% (w/w), preferably below 50 % (w/w),
and/or where the content of said one selected from the group consisting of candesartan cilexetyl, trityl candesartan cilexetyl and any intermediate of candesartan in the crude substance is below 90 wt.-%, preferably below 50 wt.-%, based on the total weight of the crude substance.

11. The process according to any of claims I to 8 for the purification of crude substance, said crude substance comprising orlistat or its intermediate lipstatin, with a resulting purity of more then 97% (w/w) in a single crystallization step and minimum resulting purity of more than 98.5% (w/w) after two consecutive crystallizations.

12. The process according to claim 11 where the purity of the crude substance comprising orlistat or its intermediate lipstatin is below 95% (w/w), preferably below 87% (w/w),
and/or where the content of orlistat or lipstatin in the crude substance is below 95 wt.-%, preferably below 87 wt.-%, based on the total weight of the crude substance.

13. The process according to any of claims 1 to 8 for the purification of crude substance comprising montelukast acid with a resulting purity of more than 97% (w/w) in a single crystallization step and a resulting purity of more then 99.0 % (w/w) after three consecutive crystallizations.

14. The process according to claim 13 where the purity of the crude substance comprising montelukast acid is below 95% (w/w), preferably below 90% (w/w), and/or
where the content of montelukast acid in the crude substance is below 95 wt.-%, preferably below 90 wt.-%, based on the total weight of the crude substance.

15. The process according to any of claims 13 and 14, **characterized in that** montelukast acid to be purified by the process is prepared by first forming a montelukast piperazine salt, preferably montelukast trans-2,5-dimethylpiperazine salt or montelukast 2-methylpiperazine salt and then converting it to montelukast acid.

## Patentansprüche

1. Kristallisationsverfahren zur Herstellung chemischer Verbindungen, insbesondere reiner chemischer Verbindungen, in welchem eine Emulsion von Tropfen in einer kontinuierlichen Phase mit einer freien Gibbs'schen Bildungsenthalpie ΔG > 0 gebildet wird, um Kristallisation und Reinigung der rohen Substanz in der kontinuierlichen oder Tropfenphase zu bewirken, wobei die Emulsion ein oberflächenaktives Mittel umfasst, **dadurch gekennzeichnet, dass** ein Animpfen mit Kristallen der reinen Substanz bei einer höheren Temperatur als die Temperatur, bei welcher die Kristallisation vollendet ist, durchgeführt wird.

2. Verfahren gemäß Anspruch 1, **dadurch gekennzeichnet, dass** die Reinheit der rohen Substanz niedriger als 90 % (w/w), bevorzugt niedriger als 80 % (w/w), am stärksten bevorzugt niedriger als 60 % (w/w) ist.

3. Verfahren gemäß einem der Ansprüche 1 oder 2, wobei die Zielendtemperatur, bei welcher die Kristallisation vollendet ist, 2-50°C unter der Temperatur beim Animpfen liegt.

4. Verfahren gemäß einem der Ansprüche 1 bis 3, wobei die Temperatur beim Animpfen 0-65°C niedriger, bevorzugt 0,5-65°C niedriger, bevorzugt 2-65°C niedriger als die Temperatur, bei welcher das gesamte Aggregatgemisch gelöst ist, ist.

5. Verfahren gemäß einem der Ansprüche 1 bis 4, wobei die mittlere Abkühlgeschwindigkeit beim Abkühlen des Kristallisationsgemisches von der Temperatur, bei welcher das Animpfen durchgeführt wird, auf die Zieltemperatur unter 1 K/min, bevorzugt unter 0,5 K/min liegt.

6. Verfahren gemäß einem der Ansprüche 1 bis 5, wobei die Emulsion einen C₁-C₅-Acetatester als eine organische Phase umfasst.

7. Verfahren gemäß einem der Ansprüche 1 bis 6, **dadurch gekennzeichnet, dass** Agglomeration von gebildeten Teilchen durch Zugabe einer zusätzlichen Menge der Emulsion zu der Suspo-Emulsion, die nach dem Animpfen und/oder während der Kristallisation vorhanden ist, hervorgerufen wird.

8. Verfahren gemäß einem der Ansprüche 1 bis 7, das zur Reinigung einer rohen Substanz verwendet wird, wobei die rohe Substanz eine umfasst, die aus der Gruppe ausgewählt ist, die aus Statinen wie Rosuvastatin, Fluvastatin, Simvastatin, Lovastatin, Atorvastatin und ihren Zwischenprodukten wie (3R,5R)-tert-Butyl-7-(2-(4-fluorphenyl)-5-isopropyl-3-phenyl-4-(phenylcarbamoyl)-1*H*-pyrrol-1-yl)-3,5-dihydroxyheptanoat; Sartanen wie Valsartan, Losartan, Candesartancilexetyl, Olmesartan, Telmisartan und Irbesartan oder ihren Zwischenprodukten wie Tritylcandesartancilexetyl; Prazolen wie Lansoprazol, Pantoprazol, Rabeprazol, Omeprazol oder Esomeprazol, Ezetimib, Fipronil, Montelukastsäure oder Montelukastsalzen wie Montelukast-2-methylpiperazinsalz oder 2-methylpiperazinsalz; Orlistat oder seinem Zwischenprodukt Lipstatin, Solifenacinbase oder Solifenacinsalzen, und Quetiapin besteht; bevorzugt eine, welche aus der Gruppe ausgewählt ist, die aus Orlistat, Tritylcandesartancilexetyl und Montelukastsäure besteht.

9. Verfahren gemäß einem der Ansprüche 1 bis 8, das zur Reinigung einer rohen Substanz verwendet wird, wobei die rohe Substanz eine umfasst, die aus der Gruppe ausgewählt ist, die aus Candesartancilexetyl, Tritylcandesartancilexetyl und jedwedem Zwischenprodukt von Candesartan besteht, mit einer Reinheit von höher als 95 % (w/w) in einem einzigen Kristallisationsschritt und einer resultierenden Reinheit von höher als 99 % (w/w) nach zwei Kristallisationsschritten.

10. Verfahren gemäß Anspruch 9, wobei die Reinheit der rohen Substanz, umfassend eine, die aus der Gruppe ausgewählt ist, die aus Candesartancilexetyl, Tritylcandesartancilexetyl und jedwedem Zwischenprodukt von Candesartan besteht, unter 90 % (w/w), bevorzugt unter 50 % (w/w) liegt,
und/oder wobei der Gehalt der einen, welche aus der Gruppe ausgewählt ist, die aus Candesartancilexetyl, Tritylcandesartancilexetyl und jedwedem Zwischenprodukt von Candesartan besteht, in der rohen Substanz unter 90 Gew.-%, bevorzugt unter 50 Gew.-% liegt, bezogen auf das Gesamtgewicht der rohen Substanz.

11. Verfahren gemäß einem der Ansprüche 1 bis 8 zur Reinigung einer rohen Substanz, wobei die rohe Substanz Orlistat oder sein Zwischenprodukt Lipstatin umfasst, mit einer resultierenden Reinheit von höher als 97 % (w/w) in einem einzigen Kristallisationsschritt und einer minimalen resultierenden Reinheit von höher als 98,5 % (w/w) nach zwei aufeinanderfolgenden Kristallisationen.

12. Verfahren gemäß Anspruch 11, wobei die Reinheit der rohen Substanz, umfassend Orlistat oder sein Zwischenprodukt Lipstatin, unter 95 % (w/w), bevorzugt unter 87 % (w/w) liegt,
und/oder wobei der Gehalt an Orlistat oder Lipstatin in der rohen Substanz unter 95 Gew.-%, bevorzugt unter 87 Gew.-% liegt, bezogen auf das Gesamtgewicht der rohen Substanz.

13. Verfahren gemäß einem der Ansprüche 1 bis 8 zur Reinigung einer rohen Substanz, umfassend Montelukastsäure, mit einer resultierenden Reinheit von höher als 97 % (w/w) in einem einzigen Kristallisationsschritt und einer resultierenden Reinheit von höher als 99,0 % (w/w) nach drei aufeinanderfolgenden Kristallisationen.

14. Verfahren gemäß Anspruch 13, wobei die Reinheit der rohen Substanz, umfassend Montelukastsäure, unter 95 % (w/w), bevorzugt unter 90 % (w/w) liegt, und/oder wobei der Gehalt an Montelukastsäure in der rohen Substanz unter 95 Gew.-%, bevorzugt unter 90 Gew.-% liegt, bezogen auf das Gesamtgewicht der rohen Substanz.

15. Verfahren gemäß einem der Ansprüche 13 und 14, **dadurch gekennzeichnet, dass** Montelukastsäure, welche durch das Verfahren gereinigt wird, durch zuerst Bilden eines Montelukastpiperazinsalzes, bevorzugt Montelukast-trans-2,5-dimethylpiperazinsalz oder Montelukast-2-methylpiperazinsalz, und dann Umwandeln davon in Montelukastsäure hergestellt wird.

## Revendications

1. Procédé de cristallisation pour la préparation de composés chimiques, en particulier de composés chimiques purs, dans lequel une émulsion de gouttelettes dans une phase continue est formée avec une enthalpie libre de Gibbs de formation ΔG > 0, pour effectuer la cristallisation et la purification de la substance brute dans la phase continue ou les gouttelettes, ladite émulsion comprenant un agent tensioactif, **caractérisé en ce que** l'on réalise un ensemencement avec des cristaux de la substance pure à une température plus élevée que la température à laquelle la cristallisation est effectuée.

2. Procédé selon la revendication 1, **caractérisé en ce que** la pureté de la substance brute est inférieure à 90% (p/p), de préférence inférieure à 80% (p/p), de manière la plus préférée, inférieure à 60% (p/p).

3. Procédé selon la revendication 1 ou 2, où la température finale ciblée, à laquelle la cristallisation est effectuée, est de 2-50°C inférieure à la température d'ensemencement.

4. Procédé selon l'une quelconque des revendications 1 à 3, où la température d'ensemencement est inférieure de 0-65°C, de préférence inférieure de 0,5-65°C, de préférence inférieure de 2-65°C à la température à laquelle tout le mélange est dissous.

5. Procédé selon l'une quelconque des revendications 1 à 4, où la vitesse moyenne de refroidissement pour le refroidissement du mélange de cristallisation depuis la température à laquelle l'ensemencement est effectué à la température ciblée est inférieure à 1 K/minute, de préférence inférieure à 0,5 K/minute.

6. Procédé selon l'une quelconque des revendications 1 à 5, où l'émulsion comprend un ester d'acétate en C₁-C₅ comme phase organique.

7. Procédé selon l'une quelconque des revendications 1 à 6, **caractérisé en ce que** l'agglomération des particules formées est induite par addition d'une quantité supplémentaire de l'émulsion à la suspo-émulsion présente après ensemencement et/ou pendant la cristallisation.

8. Procédé selon l'une quelconque des revendications 1 à 7, utilisée pour la purification d'une substance brute, ladite substance brute comprenant l'une choisie parmi le groupe consistant en les statines comme la rosuvastatine, la fluvastatine, la simvastatine, la lovastatine, l'atrovastatine et leurs intermédiaires, comme le 7-(2-(4-fluorophényl)-5-isopropyl-3-phényl-4-(phénylcarbamoyl)-1H-pyrrol-1-yl)-3,5-dihydroxyheptanoate de (3R,5R)-t-butyle ; des sartans comme le valsartan, le losartan, le candésartan cilexétil, l'olmésartan, le telmisartan et l'ibésartan ou leurs intermédiaires comme le tritylcandésartan cilexétil ; des prazoles comme le lansoprazole, le pantoprazole, le rabeprazole, l'oméprazole ou l'ésoméprazole, l'ézetimibe, le fipronil, le montélukast acide ou des sels de montélukast, comme le sel de 2-méthylpipérazine de montélukast ; l'orlistat ou son intermédiaire lipstatine, la base solifénacine ou des sels de solifénacine et la quétiapine ; de préférence, l'un choisi parmi le groupe consistant en l'orlistat, le tritylcandésartan cilexétil et le montélukast acide.

9. Procédé selon l'une quelconque des revendications 1 à 8, utilisée pour la purification d'une substance brute, ladite substance brute comprenant l'une choisie parmi le groupe consistant en le candésartan cilexétil, le tritylcandésartan cilexétil et l'un quelconque des intermédiaires du candésartan, avec une pureté de plus de 95% (p/p) en une seule étape de cristallisation et résultant en une pureté de plus de 99% (p/p) après deux étapes de cristallisation.

10. Procédé selon la revendication 9, où la pureté de la substance brute, ladite substance brute comprenant l'une choisie parmi le groupe consistant en le candésartan cilexétil, le tritylcandésartan cilexétil et l'un quelconque des intermédiaires du candésartan, est inférieure à 90% (p/p), de préférence inférieure à 50% (p/p), et/ou où la teneur en l'un des composés choisi parmi le groupe consistant en le candésartan cilexétil, le tritylcandésartan cilexétil et l'un quelconque des intermédiaires du candésartan, dans la substance brute est inférieure à 90% en poids (p/p), de préférence inférieure à 50% (p/p), sur base du poids total de la substance brute.

11. Procédé selon l'une quelconque des revendications 1 à 8, pour la purification d'une substance brute, ladite substance brute comprenant l'orlistat ou son intermédiaire lipstatine, avec une pureté résultante de plus de 97% (p/p) en une étape de cristallisation et une pureté minimale résultante de plus de 98,5% (p/p) après deux cristallisations consécutives.

12. Procédé selon la revendication 11, où la pureté de la substance brute comprenant l'orlistat ou son intermédiaire lipstatine est inférieure à 95% (p/p), de préférence inférieure à 87% (p/p), et/ou où la teneur en orlistat ou lipstatine dans la substance brute est inférieure à 95% en poids, de préférence inférieure à 87% en poids sur base du poids total de la substance brute.

13. Procédé selon l'une quelconque des revendications 1 à 8, pour la purification d'une substance brute, ladite substance brute comprenant le montélukast acide, avec une pureté résultante de plus de 97% (p/p) en une étape de cristallisation et une pureté résultante de plus de 99,0% (p/p) après trois cristallisations consécutives.

14. Procédé selon la revendication 13, où la pureté de la substance brute comprenant le montélukast acide est inférieure à 95% (p/p), de préférence inférieure à 90% (p/p), et/ou où la teneur en montélukast acide dans la substance brute est inférieure à 95% en poids, de préférence inférieure à 90% en poids sur base du poids total de la substance brute.

15. Procédé selon l'une quelconque des revendications 13 et 14, **caractérisé en ce que** le montélukast acide à purifier par le procédé est préparé en formant d'abord, un sel de pipérazine du montélukast, de préférence la sel de trans-2,5-diméthylpipérazine de montélukast ou le sel de 2-méthylpipérazine de montélukast, puis en convertissant celui-ci en montélukast acide.
